# EUROPEAN PATENT SPECIFICATION

(11) **EP 1 455 888 B1**
(45) Date of publication and mention of the grant of the patent: **29.04.2009**
(21) Application number: 02796340.4
(22) Date of filing: 28.08.2002
(51) Int. Cl.: A61M 35/00

(54) **TOPICAL ADMINISTRATION DEVICE**
VORRICHTUNG ZUR TOPISCHEN VERABREICHUNG
DISPOSITIF D'ADMINISTRATION TOPIQUE

(30) Priority: 29.08.2001 US 315894 P
(43) Date of publication of application: 15.09.2004
(73) Proprietor: PharmaKodex Limited, Chippenham Wiltshire SN14 6FH (GB)
(72) Inventor: TOBYN, Michael John, Wilts BA14 9SS (GB); STANIFORTH, John Nicolas, Bath Banes BA 2 7AT (GB); MILLS, Sharon Ann, Bath Banes BA3 5UX (GB); PATERSON, Graeme Lindsay Jonathan, Cheddar, Somerset BS27 3HX (GB); ALTHORPE, Christopher, Penylan Cardiff CF23 5DU (GB)
(74) Representative: Teuten, Andrew John
(86) International application number: PCT/IB2002/003485
(87) International publication number: WO 2003/018102

(56) References cited:
- EP-B- 1 383 405
- EP-B- 1 383 405
- WO-A-95/34340
- DE-A- 2 917 805
- US-A- 5 827 235
- US-B1- 6 224 572

## Description

### FIELD OF INVENTION

The present invention is directed to a dosing device, which can be utilized to meter and administer a pharmaceutical formulation to the skin of a mammal, e.g., humans, and a method for preparing a dosing system.

### BACKGROUND OF THE INVENTION

Drug therapy prescribed by a health care professional typically includes the selection of drug, the potency or strength of the drug and the appropriate dosing interval. Most pharmaceutical formulations, e.g., tablets, capsules and liquids can meet these requirements as a patient can take a unit dose of the prescribed drug which has been precalibrated to provide an indicated strength. An oral liquid formulation when used correctly gives even more precision as the liquid can be measured with a standard measurer in order to obtain precise individual doses of drug. When a prescriber diagnosis a condition and prescribes a topical treatment such as a cream, ointment, lotion, liquid, etc, precision dosing is more difficult.

Creams and ointments are typically packages in a tube or jar and exact dosing of drug cannot be calibrated by the patient. In fact, many times, the prescriber simply instructs the patient to apply a particular formulation a certain number of times daily, e.g. twice daily, and does not provide the patient with any insight as to what would be too little of a dose (subtherapeutic) or what would be too much (possibly resulting in increased side effects).

Another problem associated with topical pharmaceutical formulations is that when a topical formulation is dispensed from a tube, the subsequent administration is typically by the patient or caregiver taking the formulation in their hand and applying it to the affected area. This method has many undesirable consequences. For example, the amount dispensed from the tube is not the amount, which will be administered to the intended site of action. This is due to the fact that an amount of the formulation will be absorbed into the skin of the hand of the patient or the caregiver. Therefore, even assuming that the patient fortuitously dispenses a proper amount of formulation, a subtherapeutic dose may be applied due to the amount absorbed by the skin. This can be avoided by, e.g., the patient wearing a latex glove during application. This is often objectionable for many reasons. Many people find the feel of latex gloves unacceptable and uncomfortable. Also, many people are allergic to latex and the use of such an administration aid can precipitate anaphylactic shock. Further, latex gloves add additional expense to drug therapy and may not be readily available to all patients.

Another problem of the hand administration method is that drug can be absorbed to an area that is not intended to be. For example, if the hand is used to apply formulation prescribed for the torso, drug will also be absorbed by the hand. This can be a problem with drugs which have high toxicity or produce undesirable side effects. Another issue with this common form of administration is that many patients do not take appropriate measure to clean and sanitize their hands prior to administration which can lead to spread of microbes.

The prior art is replete with specific examples of topical formulations where specific dosing regimens, and/or particular maximum dosages, have been required. For example, doxepin hydrochloride, a systemic antidepressant agent, is recommended to be applied thinly three to four times daily, typically with a maximum 3g administration per application, a typical daily maximum administration of 12g and suitably coverage should be less than about 10% of body surface.

Clobetasol propionate, a steroidal anti-inflammatory agent is recommended to be applied thinly one to two times daily for up to four weeks, typically with a maximum administration of about 50g of a 0.05% preparation per week.

Diflucortolone valerate, another anti-inflammatory agent is recommended to be applied one to two times daily for up to four weeks (0.1% preparation) or two weeks (0.3% preparation), with typically a maximum administration of about 60g of a 0.3% preparation per week.

Monitoring the maximum dosage has been especially important for topical formulations of calcipotriol. There has been seen to be a risk of hypercalcemia if the recommended maximum weekly dose of calcipotriol has been exceeded. The risk of hypercalcemia and methods to avoid this risk have not always been clearly explained in patient information provided with topical formulations of calcipotriol. For example, the package insert for the topical formulation of calcipotriol available under the trademark Dovonex, advises liberal application of the formulation despite the above described disadvantage of possible hypercalcemia with excessive dosing. The recommended dosing regime for a topical formulation of calcipotriol, however, has been to apply once or twice daily, with a maximum weekly dose of 100g. For patients over six years of age, the formulation should be applied twice daily; for patients from six to twelve years, a maximum weekly dose of 50g; and for patents over twelve years, a maximum weekly dose of 75g.

To alleviate problems encountered with treatment regimes where it has been important to observe a maximum dosage of a therapeutic agent for topical administration, it would be beneficial to be able to provide means for accurately administering a therapeutic agent and applying it directly to the skin of a patient without the need for intermediary manipulation by the patient or the caregiver. Such accurate administration should obviate the detrimental side effects that have hitherto been observed.

With respect to the inaccuracy of dosing associated with typical topical formulation tubes, one method of metering, or dosing, the amount of a therapeutic agent applied to a patient's skin in such a topical formulation has been for a patient to squeeze such a topical formulation from a dispenser, such as a tube, along an index finger starting at the fingertip down to the first joint and the amount of therapeutic agent thus to be administered has been known as the fingertip unit (FTU). One FTU generally approximates to about 500mg of a topical formulation and is generally sufficient to cover an area that is twice that of a flat adult hand. Such administration has not, however, hitherto achieved accurate dosing. In particular, a significant disadvantage associated with the FLU is that it is only an approximate unit and its magnitude varies from patient to patient.

Another method, as in the case of nitroglycerin paste, is to measure the paste on a calibrated paper supplied by the manufacturer. This is also not accurate as the width of the paste being measured can significantly alter the measurement. For example, if the patient dispenses the ointment slowly, a wider mass will be measured longitudinally, which would result in an overdose. Conversely, if the patient dispenses over the measured area quickly, the width of the paste being measured may be thinner and a subtherapeutic dose may result.

It has also been known to deliver therapeutic agents transdermally by applying to the skin of a patient an adhesive patch containing a therapeutic agent. Such patches have typically further included a rate-moderating membrane, an adhesive, a liner and a backing material. The adhesive has often required special formulation to ensure compatibility with the other components of such patches and this type of formulation has often increased the cost of such patches. Furthermore, not all therapeutic agents are suitable for inclusion in such patches for many reasons, such as stability, absorption, etc.

WO 95/34340 discloses an apparatus for storing a viscous substance and for applying it onto the skin. The apparatus comprises a reservoir part and an applicator part.

WO 02/087384 discloses a dispenser for applying a material to a surface. The material is dispensed as a result of the operation of a piston. In some embodiments of the device an opening is provided, through which a cream or gel is forced upon actuation of the device. Alternatively, the material is formulated as a stick, which is applied via erosion at a face of the stick.

Due to the disadvantages of topical pharmaceutical formulations discussed above, there exists a need in the art for the development of a device and method which address both the problem of inaccurate dosing and the problem of administration as discussed above.

### OBJECTS AND SUMMARY OF THE INVENTION

It is an object of the present invention to provide a dosing device for the topical administration of a unit dose of a pharmaceutical formulation

It is a further object of certain embodiments of the invention to provide a dosing device for containing a supply of multiple unit doses of pharmaceutical formulation, the device capable of metering an accurate unit dose of the pharmaceutical formulation.

It is a further object of certain embodiments of the invention to provide a dosing device for containing a single unit dose of pharmaceutical formulation, the device capable of dispensing and applying the unit dose onto the skin of a patient.

It is a further object of certain embodiments of the invention to provide a dosing device for containing a supply of multiple unit doses of pharmaceutical formulation, the device capable of dispensing and applying the formulation onto the skin of a patient.

It is a further object of certain embodiments of the invention to provide a dosing device for containing a supply of multiple unit doses of pharmaceutical formulation, the device capable of metering an accurate unit dose of the pharmaceutical formulation and applying the unit dose onto the skin of a patient.

It is a further objet of certain embodiments of the invention to provide a dosing device for containing a supply of multiple unit doses of pharmaceutical formulation, the device capable of metering an accurate unit dose of the pharmaceutical formulation and applying the unit dose onto the skin of a patient wherein the device can be operated with one hand.

It is a further object of certain embodiments of the invention to provide a dosing device for containing a supply of multiple unit doses of pharmaceutical formulation, the device capable of metering an accurate unit dose of the pharmaceutical formulation and applying the unit dose onto the skin of a patient without the need for the patient or caregiver having to apply the drug manually or with an intermediate receptacle.

It is a further object of certain embodiments of the invention to provide a dosing device for containing a supply of multiple unit doses of pharmaceutical formulations, the device capable of use by a caregiver without exposing themselves to the drug, e.g., their hands.

It is a further object of certain embodiments of the invention to provide a dosing device for containing a supply of multiple unit doses of pharmaceutical formulations, the device capable of use by a patient without exposing themselves to the drug at an undesired location, e.g., their hands.

The above objects of the invention and others, can be achieved by virtue of the present invention which is directed to a dosing device encoding to claim 1.

In preferred embodiments, the invention provides a dosing system comprising a pharmaceutical formulation comprising a drug and a carrier suitable for topical application contained in a dosing device according to claim 1.

There is also described to a method for topically administering a pharmaceutical formulation to the skin of a mammal, the method comprising (i) actuating a dosing device for topically administering a pharmaceutical formulation to the skin of a mammal, the device comprising a housing storing at least one unit dose of a pharmaceutical formulation comprising a drug incorporated with a pharmaceutically acceptable carrier suitable for topical application onto the skin of said mammal; an applicator adapted for topically administering a unit dose of the pharmaceutical formulation directly onto the skin; and an actuator capable of metering a single unit dose of the pharmaceutical formulation from a first position in which the unit dose is stored in the housing to a second position in which the single unit dose is external to the device on the applicator so that the single unit dose can be topically administered; and (ii) applying the unit dose directly onto the skin of a mammal with the applicator.

In other preferred embodiments, the invention is directed to a method of preparing a dosing system for topical delivery of a pharmaceutical formulation including (i) preparing at least one unit dose of a pharmaceutical preparation comprising a drug incorporated with a pharmaceutically acceptable carrier suitable for topical application onto the skin of said mammal; and (ii) placing the at least one unit dose into a dosing device according to claim 1. The term "semi-solid" for purposes of the present invention includes ointments, gels, emulsion, mousse, magmas, milks, pastes, creams and foams. In certain preferred embodiments of the present invention, the semi-solid is an ointment, cream or gel.

For purposes of the present invention, the term "device" refers to an apparatus capable of delivering at least one unit dose of drug.

The term "system" refers to a drug delivery device in combination with a pharmaceutical formulation for topical delivery.

The term "therapeutic agent" or "drug" as used herein denotes any active substance suitable to be topically administered to a mammal, e.g., humans) for a therapeutic or prophylactic purpose and being suitable for use in any formulation in connection with the present invention. The term "therapeutic agent" as used herein also includes any pharmaceutically acceptable equivalent of the active substance, such as a pharmaceutically acceptable salt, ester, prodrug or metabolite thereof. Isomers of all disclosed agents are also encompassed by this disclosure.

The term "unitary" when used with respect to the device of the present invention means that the applicator and the housing are in a fixed position and do not have to be removed from each other to apply the unit dose of formulation from the applicator, or have their orientation with respect to each other altered in order to apply the dose. It is preferred that the spatial relationship between the housing and the applicator are the same before, during, and after actuation and subsequent application.

The terms "topically administered" or "topical administration" as used herein includes (i) administration of a therapeutic agent suitable for use in the present invention for local treatment on the surface of the skin; (ii) administration of a therapeutic agent which is absorbed to provide a local effect in the region of application (e.g., in the muscle or tissue at or near the point of administration; and (iii) administration of a therapeutic agent suitable for use in the present invention for non-local treatment by administration through the skin, in other words for administration into the blood stream of a mammal for systemic treatment.

The term "treatment" as used herein denotes the treatment of established conditions as well as the prophylaxis thereof. The precise treatment conditions for any pharmaceutical formulation, product or method according to the present invention will of course depend on the precise nature of a condition being treated, the age and sex of the patient and will ultimately be at the discretion of an attendant physician.

The term "drug" refers to any agent which is capable of providing a therapeutic effect to a patient

The term "dispense", when used in connection with the devices and systems of the present invention, means that the device or system delivers a unit dose contained in the housing of the device to the applicator, external from the device.

The term "administer", when used in connection with the devices and systems of the present invention, means that the device applies the unit dose directly onto the skin

The term "patient" refers to humans as well as other mammals in need of a topical therapeutic agent, e.g., household pets or livestock. This term also refers to humans or mammals in need of or receiving prophylactic treatment.

The term "unit dose" means a formulation suitable for single administration which contains an effective amount of an agent to be administered.

### BRIEF DESCRIPTION OF THE DRAWINGS

Fig. 1 is a view of one embodiment of a dosing device according to the present invention.
Fig. 2 is a section view of the embodiment shown in Fig. 1.
Fig. 3 is an exploded view of the components of the embodiment shown in Fig. 1.
Fig. 4a is a partial section view of the embodiment shown in Fig. 1 showing the applicator in a closed position.
Fig. 4b is a partial section view of the embodiment shown in Fig. 1 showing the applicator in an open position.
Figs. 5a, 5b, and 5c are perspective view of a button mechanism of the embodiment shown in Fig. 1.
Fig. 6 is a perspective view of a button non-return mechanism of the embodiment shown in Fig. 1.

### DETAILED DESCRIPTION

The present invention relates to a dosing device for topically administering a pharmaceutical formulation directly to the skin of a mammal. The dosing device of the present invention includes a housing capable of holding at least one unit dose of a pharmaceutical formulation comprising a drug and a suitable carrier therefor, an applicator adapted for topically administering the unit dose of a pharmaceutical formulation directly onto the skin, and an actuator. When the dosing device is actuated, the device can meter a unit dose of the pharmaceutical formulation from the housing to the applicator.

The dosing device of the invention can be used to apply a pharmaceutical formulation directly to the skin of a mammal e.g., as a semi-solid or liquid pharmaceutical formulation. Certain embodiments of the invention are adapted to contain and meter semi-solid pharmaceutical formulations such as an ointment, gel, emulsion, lotion, spray, cream or paste and certain embodiments are adapted to contain and meter a liquid such as a suspension or solution.

The pharmaceutical formulation can be placed in the housing of the dosing device, wherefrom unit doses can be metered directly to the applicator and administered to the skin according to a dosing schedule either by the patient or the caregiver. The size of the unit dose is dependent on the amount of drug to be provided for the intended therapeutic effect and the amount of the pharmaceutically acceptable carrier medium. Typically, a unit dose from about 0.10 grams to about 5 grams can be metered from the housing to the applicator and would be sufficient to contain a therapeutically effective amount of the drug to be delivered. However, this range is not limiting and can be smaller or higher, depending on the amount or potency of drug and carrier that is necessary. Additional unit doses can be delivered from the housing to the applicator upon a subsequent actuation of the actuator until depletion of the pharmaceutical formulation from the housing.

In order to promote patient compliance, certain embodiments of the invention include a counter which indicates the number of doses actuated. Alternatively, the dosing device can include an indicator to display the number of doses remaining in the dosing device. The ability to count remaining doses is useful especially to a patient who may have forgotten if a previous dose has been taken. A counter also minimizes the likelihood of the patient miscounting the proper dosage and taking a double dose or skipping a dose due. The counter will also keep the user apprized as to when the drug will run out and will help to improve patient compliance by allowing for proper planning for the patient to frequent a pharmacy in a timely manner. This can reduce the likelihood of a patient being "surprised" when the system does not provide any unit doses. The device can alternatively count the doses delivered by counting up, or can count down to show the number of unit doses remaining in the system. The counter can be an electrical or mechanical mechanism which are commonly known in the art. The indicator can also be a visual mechanism, e.g., the topical formulation could fall below a colored marker which would indicate the number of doses remaining, the device can expose the internal formulation to view in a window, or other mechanisms known in the art.

In certain embodiments, after depletion or partial depletion of the at least one unit dose, the dosing device can optionally be reloaded with at least one additional unit dose. Alternatively, if the dosing device is not capable of being reloaded, then the device is disposable. This embodiment is beneficial for many reasons. Most prominently, a disposable device will give a patient, the prescriber and the manufacturer greater assurances that the patient is receiving a proper dosage from a dosing system that has not been subject to improper handling an/or internal handling for a long duration of time. Such disposable devices may also reduce the overall cost of manufacture, as the device would only have to be manufactured to provide an accurate dosage for a finite period of time.

The housing of the device is preferably capable of containing multiple doses of the pharmaceutical formulation in order to provide a multiplicity of unit doses. The number of unit doses contained in the housing of the device and capable of being delivered onto the skin depends on, among other factors, the frequency of dosing and the duration of therapy of the drug to be dispensed. Preferably, the housing of the dosing device can hold from about 5 unit doses to about 400 unit doses of the pharmaceutical formulation. More preferably, the housing of the dosing device is adapted to contain from about 40 doses to 120 unit doses of the pharmaceutical formulation. In certain aspects of the invention, the housing of the dosing device is preferably adapted to contain at least 2 unit doses, and more preferably at least 5 unit doses of the pharmaceutical formulation. In other aspects, the housing of the dosing device can preferably contain 30 unit doses, and more preferably 365 unit doses.

In certain embodiments, the dosing device can contain multiple dosing mechanisms in order to provide dosage amounts for different times. For example, the system can comprise two dosing mechanisms which can provide a different dosage amount in the morning and the evening.

In certain embodiments, the dosing device of the invention can include more than one housing, each containing a different drug or pharmaceutical formulation. Upon actuation, the desired amount of each drug is metered out for delivery to the skin at the same time or sequentially as desired. Dosing devices containing multiple housings or multiple reservoirs in a housing would be beneficial for combination therapy, would eliminate the need for multiple devices and would allow a much wider range of possible doses and dose combinations.

In certain embodiments, the system of the invention can be configured wherein the housing is replaceable, e.g., in the form of a replaceable cartridge, or wherein the housing is capable of being refilled, e.g., by including a removable plug wherein bulk topical formulation can be introduced. However, in embodiments wherein the housing is capable of being refilled, it is preferable that a replaceable housing is utilized such as the previously disclosed cartridge device rather than refilling the housing with a bulk formulation through an unplugged hole as the latter may be more prone to human error, e.g., loss of product due to spilling or improper manipulation. Further, the handling of bulk topical formulation may result in contamination of the device, formulation or both, with moisture and/or contaminants.

In certain preferred embodiments, the dosing device is ergonomically engineered to facilitate a caregiver to administer a topical pharmaceutical formulation to the skin of a patient and in other embodiments the dosing device is ergonomically engineered to facilitate self-administration. Preferably, the dosing device is ergonomically engineered to facilitate both situations.

In certain preferred embodiments, the dosing device of the invention could include a means for preventing the actuator from functioning after a predetermined number of actuation, for the predetermined time period. The means controlling the function of the actuator can be mechanical or electrical means as known in the art. The dosing device of the invention can be configured such that the desired time period is the dosing interval of the drug. For example, in certain preferred embodiments, the dosing interval of the dosing device is from about one hour to about twenty-four hours, more preferably from about four (4) hours to about twelve (12) hours. In certain embodiments, the dosing device is engineered such that the predetermined number of actuations is a number which administers the prescribed amount of a pharmaceutical formulation, preferably such that the predetermined number of actuations is one actuation or more preferably is more than one actuation. Moreover, in order to prevent accidental actuation during application of the dosing device to the skin, in certain aspects, the desired time period for preventing the actuator from functioning is at least the time needed to administer topically the previously metered unit dose.

In certain embodiments, the pharmaceutical composition included in the device does not exceed a 10% overage, preferably does not exceed a 5% overage and most preferably does not require any overage. In situations where there is overage, the device can be configured with a mechanism which prevents the patient from accessing the overage. This could prevent the patient from being administered a partial dose which may be subtherapeutic. This feature can also prevent a patient from being administered more unit doses than prescribed by the physician.

It is important that the dosing device of the invention provides accurate and reproducible unit doses. Accordingly, in certain embodiments, each unit dose metered from the device does not vary by more than 10% throughout the life of the device at room and other temperature ranges. The temperature range may vary from 0°C to less than 100°C, more preferably from about 10°C to about 80°C, and most preferably from about 20°C to about 40°C. In certain other embodiments, each unit dose metered from the dosing device does not vary by more than 5% at a temperature range from about 0°C to less than 100°C, more preferably from about 10°C to about 80°C, most preferably from about 20°C to about 40°C including room temperature. In yet another aspect of the invention, the dosing device is capable of metering each unit dose from the device such that it does not vary by more than 1% at a temperature range from about 0°C to less than 100°C, more preferably from about 10°C to about 80°C, most preferably from about 20°C to about 40°C, including room temperature.

In most preferred embodiments, the dosing device of the invention provides a direct contact with the skin of a mammal. Such direct contact can be accomplished by providing the dosing device with an applicator having a surface that can easily come in direct contact with the skin of the mammal. Applicators that can accomplish this goal include those having a surface that can be either flat or convex, smooth or ridged. Moreover, when the device is held upright, the flat surface is preferably angled from a base line perpendicular to the dosing device. For example, a convex applicator surface can be provided by a roller ball placed in the applicator such that the unit dose can be rolled on the skin. In another aspect of the invention, the applicator can include a static surface rigidly connected with the housing and adapted to spread the dose onto the skin.

In certain embodiments, the applicator is preferably made from the material which inhibits microbial proliferation and/or is a non-wetting material which promotes the formation of droplets when exposed to moisture. In certain preferred embodiments, both the housing and the applicator of the dosing device are preferably comprised of a material which inhibits microbial proliferation, such as for example a silver containing plastic. Moreover, in certain other preferred embodiments, both the applicator and the housing of the dosing device are preferably comprised of a non-wetting material which promotes the formation of droplets when exposed to moisture, such as for example, silicone. The actuator can also be manufactured of the materials disclosed above

In other preferred embodiments, in order to prevent the decomposition of certain pharmaceutical formulations, the housing can be comprised of an aluminum lining or a plastic coated with aluminum. The applicator and/or the actuator can also be comprised of this material.

The dosing device of the present invention should contain the supply of pharmaceutical formulation with a tight seal from the external environment (e.g. from air, moisture and water) to provide many benefits. Such a configuration minimizes contamination of the contained formulation by contaminants and microbes. A tight seal also allows the device to be cleaned by a solvent, preferably water, without the formulation coming in contact with any of the liquid. Introduction of a liquid such as water into a semisolid formulation may hinder the accurate metering of the formulation if the device is adapted to deliver semisolids and the semisolid loses viscosity from the introduction of liquid. Preferably, the device of the present invention can be submerged in water for at least 30 seconds without consequence. Other embodiments can be submerged for a longer period, e.g., at least 2 hours, without consequence.

According to the invention, the applicator of the dosing device includes a valve disposed in an opening of the housing wherein upon actuation, the valve is movable between an open position to allow discharge of the unit dose through the opening to the applicator and a closed position to seal the opening. In certain aspects, the actuation of the actuator can cause a positive pressure in the housing of the device, which positive pressure can cause the valve to move from a closed position to an open position.

In certain other embodiments, the actuator comprises a button which upon actuation can cause the unit dose to be discharged from the housing to the applicator. Preferably, the button is positioned on the device to allow a user to actuate and then apply the unit dose to the skin with one hand. In preferred embodiments, the user does not have to reposition the hand from an actuation position to an application position and all steps of actuation and application can be performed with minimal or no repositioning of the hand.

In other embodiments, the actuator (preferably a button) is flush with the surface of the device or can be recessed. This minimizes the accidental actuation of an additional unit dose during the Application process. In such embodiment, the actuator can be covered by the hand during application and will not discharge an undesired unit dose.

An actuator useful for the device of the invention can also comprise other types of mechanism for dispensing unit doses from the housing to the applicator. For example, the actuator can comprise a button, a rack, a pinion, and a lead screw in operative connection with each other, and the wherein actuation of the button causes the unit dose to be discharged from the housing to the applicator. Preferably, the dosing device further comprises a protective cover adapted to cover the valve and applicator in a closed position. A spring mechanism can also be used to move the valve from are open to a closed position.

In certain embodiments, the button can be moveable between a non-actuated position and an actuated position. When the actuation mechanism includes a lead screw, then the lead screw may preferably include a ratchet logic adapted to reduce the back pressure in the container. The lead screw may further comprise a valve logic for moving the valve from a closed position to an open position.

To avoid delivering a partial dose and/or contaminating the device, preferably, the dosing device of the invention further comprises a non-return mechanism adapted to prevent the actuator from delivering a partial dose and/or contaminating the device. Moreover, the actuator can also be adapted to substantially prevent air from entering the house during or after actuation.

In certain embodiments, the liquid contained in the device can be converted into a foam during the actuation process. Advantageously, the formulations to be included in the present invention can be formulated wherein the drug is substantially absorbed by the skin (e.g., 95% or more) over a period of less than about 30 minutes after administration, less than about 20 minutes after administration, or less than about 5 minutes after administration. In other embodiments, the dosing system of the invention provides for topical application of a pharmaceutical formulation wherein about 50% of the drug contained in the pharmaceutical formulation is absorbed by the skin over a period of more than about twelve (12) hours after administration, more than about six (6) hours after administration, or more than about two (2) hours after administration.

Therapeutic agents which can be used with the dosing system of the invention include all drugs which can be delivered on or through the skin for either a local or systemic effect.

These compounds include drugs in all of the major therapeutic areas, including, but not limited to, ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha-two-adrenergic agonists, analgesics, antipyretics and anti-inflammatory agents, androgens, local and general anesthetics, antiaddictive agents, antiandrogens, antiarrhythmic agents, antiasthmatic agents, anticholinergic agents, anticholinesterase agents, anticoagulants, antidiabetic agents, antidiarrheal agents, antidiuretic, antiemetic and prokinetic agents, antiepileptic agents, antiestrogens, antifungal agents, antihypertensive agents, antimicrobial agents, antimigraine agents, antimuscarinic agents, antineoplastic agents, antiparasitic agents, antiparkinson's agents, antiplatelet agents, antiprogestins, antithyroid agents, antitussives, antiviral agents, atypical antidepressants, azaspirodecanediones, barbituates, benzodiazepines, benzothiadiazides, beta-adrenergic agonists, beta-adrenergic antagonists, selective beta-one-adrenergic antagonists, selective beta-two-adrenergic agonists, bile salts, agents affecting volume and composition of body fluids, butyrophenones, agents affecting calcification, calcium channel blockers, cardiovascular drugs, catecholamines and sympathomimetic drugs, cholinergic agonists, cholinesterase reactivators, dermatological agents, diphenylbutylpiperidines, diuretics, ergot alkaloids, estrogens, ganglionic blocking agents, ganglionic stimulating agents, hydantoins, agents for control of gastric acidity and treatment of peptic ulcers, hematopoietic agents, histamines, histamine antagonists, 5-hydroxytryptamine antagonists, drugs for the treatment of hyperlipoproteinemia, hypnotics and sedatives, immunosupressive agents, laxatives, methylxanthines, monoamine oxidase inhibitors, neuromuscular blocking agents, organic nitrates, pancreatic enzymes, phenothiazines, progestins, prostaglandins, agents for the treatment of psychiatric disorders, retinoids, sodium channel blockers, agents for spasticity and acute muscle spasms, succinimides, thioxanthines, thrombolytic agents, thyroid agents, tricyclic antidepressants, inhibitors of tubular transport of organic compounds, drugs affecting uterine motility, vasodilators, vitamins and any therapeutically effective combinations thereof.

Representative drugs include, by way of example but not limited to, bepridil, diltiazem, felodipine, isradipine, nicardipine, nifedipine, nimodipine, nitredipine, verapamil, dobutamine, isoproterenol, carterolol, labetalol, levobunolol, nadolol, penbutolol; pindolol, propranolol, sotalol, timolol, acebutolol, atenolol, betaxolol, esmolol, metoprolol, albuterol, bitolterol, isoetharine, metaproterenol, pirbuterol, ritodrine, terbutaline, alclometasone, aldosterone, amcinonide, beclomethasone, dipropionate, betamethasone, clobetasol, clocortolone, cortisol, cortisone, corticosterone, desonide, desoximetasone, 11-desoxycorticosterone, 11-desoxycortisol, dexamethasone, diflorasone, fludrocortisone, flunisolide, fluocinolone, fluocinonide, fluorometholone, flurandrenolide, halcinonide, hydrocortisone, medrysone, 6.alpha.-methylprednisolone, mometasone, paramethasone, prednisolone, prednisone, tetrahydrocortisol, triamcinolone, benoxinate, benzocaine, bupivacaine, chloroprocaine, cocaine, dibucaine, dyclonine, etidocaine, lidocaine, mepivacaine, pramoxine, prilocaine, procaine, proparacaine, tetracaine, alfentanil, choroform, clonidine, cyclopropane, desflurane, diethyl ether, droperidol, enflurane, etomidate, halothane, isoflurane, ketamine hydrochloride, meperidine, methohexital, methoxyflurane, morphine, propofol, sevoflurane, thiamylal, thiopental, acetaminophen, allopurinol, apazone, aspirin, auranofin, aurothioglucose, colchicine, diclofenac, diflunisal, etodolac, fenoprofen, flurbiprofen, gold sodium thiomalate, ibuprofen, indomethacin, ketoprofen, meclofenamate, mefenamic acid, meselamine, methyl salicylate, nabumetone, naproxen, oxyphenbutazone, phenacetin, phenylbutazone, piroxicam, salicylamide, salicylate, salicylic acid, salsalate, sulfasalazine, sulindac, tolmetin, acetophenazine, chlorpromazine, fluphenazine, mesoridazine, perphenazine, thioridazine, trifluorperazine, triflupromazine, disopyramide, encainide, flecainide, indecainide, mexiletine, moricizine, phenytoin, procainamide, propafenone, quinidine, tocainide, cisapride, domperidone, dronabinol, haloperidol, metoclopramide, nabilone, prochlorperazine, promethazine, thiethylperazine, trimethobenzamide, buprenorphine, butorphanol, dezocine, diphenoxylate, drocode, hydrocodone, hydromorphone, levallorphan, levorphanol, loperamide, meptazinol, methadone, nalbuphine, nalmefene, nalorphine, naloxone, naltrexone, oxybutynin, pentazocine, isosorbide dinitrate, nitroglycerin, theophylline, phenylephrine, ephidrine, pilocarpine, furosemide, tetracycline, chlorpheniramine, ketorolac, bromocriptine, guanabenz, prazosin, doxazosin, flufenamic acid, pharmaceutically acceptable salts thereof and any therapeuctically effective combinations.

Other representative drugs useful with the dosing system the invention include without limitation, benzodiazepines, such as alprazolam, brotizolam, chlordiazepoxide, clobazam, clonazepam, clorazepate, demoxepam, diazepam, flumazenil, flurazepam, halazepam, lorazepam, midazolam, nitrazepam, nordazepam, oxazepam, prazepam, quazepam, temazepam, triazolam, and the like; an antimuscarinic agent such as anisotropine, atropine, clidinium cyclopentolate, dicyclomine, flavoxate, glycopyrrolate, hexocyclium, homatropine, ipratropium, isopropamide, mepenzolate, methantheline, oxyphencyclimine, pirenzepine, propantheline, scopolamine, telenzepine, tridihexethyl, tropicamide, and the like; an estrogen such as chlorotrianisene, siethylstilbestrol, methyl estradiol, estrone, estrone sodium sulfate, estropipate, mestranol, quinestrol, sodium equilin sulfate, 17.beta.-estradiol (or estradiol), semi-synthetic estrogen derivatives such as the esters of natural estrogen, such as estradiol-17.beta.-enanthate, estradiol-17.beta.-valerate, estradiol-3-benzoate, estradiol-17.beta.-undecenoate, estradiol 16,17-hemisuccinate or estradiol-17.beta.-cypionate, and the 17-alkylated estrogens, such as ethinyl estradiol, ethinyl estradiol-3-isopropylsulphonate, and the like; an androgen such as danazol, fluoxymesterone, methandrostenolone, methyltestosterone, nandrolone decanoate, nandrolone phenpropionate, oxandrolone, oxymetholone, stanozolol, testolactone, testosterone, testosterone cypionate, testosterone enanthate, testosterone propionate, and the like; or a progestin such as ethynodiol diacetate, gestodene, hydroxyprogesterone caproate, levonorgestrel, medroxyprogesterone acetate, megestrol acetate, norethindrone, norethindrone acetate, norethynodrel, norgestrel, progesterone, pharmaceutically acceptable salts thereof and any therapeutically effective combinations thereof.

Therapeutic agents having local activity which can be used with the dosing system of the invention include, for example, active substances for use in the treatment of disorders of the skin, such disorders including, by way of example, psoriasis, eczema, acne, nappy rash, other inflammatory disorders, bacterial infections, viral infections, fungal infections, anaphylactic conditions, malignancies and warts.

Advantageously, therapeutic agents having local activity for use with the dosing system of the invention can be selected from the group consisting of local anesthetics, corticosteroids, antibacterial agents, antifungal agents or any therapeutically effective combination thereof.

More particularly, therapeutic agents having local activity for use with the system of the invention can be selected from the group consisting of tetracaine, benzocaine, lindocaine, hydrocortisone, beclomethasone diproprionate, clobetasol proprionate, fluticasone proprionate, ichthammol, lithium succinate, coal tar, dithranol, benzoyl peroxide, tretinoin, sulphur, vitamin D and derivatives thereof, framycetin, chlortetracycline hydrochloride, fusidic acid, clotrimazole, econazole, amorolfine and terbenafine, or any therapeutically effective combination thereof.

Local anesthetics include without limitation an anesthetic selected from the group consisting of bupivacaine, levo-bupivacaine, ropivacaine, benzocaine, dibucaine, procaine, chloroprocaine, prilocaine, mepivacaine, etidocaine, tetracaine, lidocaine, and xylocaine, as well as anesthetically active derivatives, analogs, isomers and mixtures thereof.

In embodiments where the therapeutic drugs produce a local effect, active agents include without limitation antiviral agents (e.g., acyclovir and idoxuridine, etc.), antifungal agents (e.g., amphotericin B, clotrimazole, nystatin, ketoconazole, miconazole, butocouazole, haloprogin, etc.), antibiotic agents (penicillins, cephalosporins erythromycin, tetracycline, clindamycin, aminoglycosides, chloramphenicol, polymixin b, bacitracin, neomycin, gentamycin etc.), antiseptics (e.g., povidone-iodine, methylbenzethonium chloride, etc.), antiparasitics (e.g., lindane, anthralin, etc.) analgesic agents (e.g., methylsalicylate, salicylic acid, dyclonine, aloe vera etc.), local anesthetics (e.g., benzocaine, lidocaine, xylocaine, butamben picrate, etc.), anti-inflammatory agents (e.g., steroidal compounds such as dexamethasone, betamethasone, prednisone, prednisolone, triamcinolone, hydrocortisone, alclometasone, amcinonide, diflorasone, etc. as well as non-steroidal anti-inflammatories ), anti-itch and irritation-reducing compounds (e.g., antihistamines such as diphenhydramine and psoriasis treatments) ; bum relief compounds (e.g., o-amino-p-toluenesulfonamide, monoacetate, etc.); depigmenting agents (e.g., monobenzone); and hormonal agents (e.g., oestriol).

In certain embodiments of the invention, the drug included in the pharmaceutical formulation comprises a pharmaceutically acceptable source of nitrites. In certain embodiments, the nitrites are included with a pharmaceutically acceptable acidifying agent as disclosed in WO 95/22335. In other embodiments, the drug can include a composition comprising an aqueous solution of nitric acid and nitrous acid as disclosed in U.S. Patent No. 4,595,591. In other embodiments, the composition can comprise a vaso-active composition comprising nitrogen oxide generated from an admixture of ferrous sulphate, an organic acid and an inorganic nitrite as disclosed in U.S. Patent No. 5,648,101. In other embodiments the nitrogen oxide can be used to inhibit viruses as disclosed in WO 96/02268. In other embodiments, the composition can comprise nitrous oxide in combination with a fatty acid or a lower alkyl ester thereof as disclosed in WO 93/25213.

In other embodiments comprising nitrogen oxides, the nitrogen oxide is produced when a pharmaceutically acceptable acidifying agent and a pharmaceutically acceptable donor of nitrogen oxides or precursor thereof are brought into contact at the site of action as disclosed in WO 99/44622.

In certain embodiments of the invention, the device can comprise two housings, each containing a separate drug. This would be useful where it is desirable to have two formulations dispensed simultaneously as in WO 99/44622. The two formulations can both be dispensed upon actuation where they mix upon movement to the applicator. In other embodiments, the formulations can be actuated separately and then administered to the patient sequentially and mixed upon application of the second formulation.

In other embodiments, the formulation can contain a permeation enhancer which is known in the art to improve the absorption of the drug. Such permeation enhancers are disclosed in WO 99/24036.

In other embodiments intended for local delivery, the pharmaceutical composition can comprise zinc ions which improve efficacy by enhancing skin penetration and reduce the risk of side effects by discouraging the passage of the drugs through the skin to the underlying systemic circulation.

Pharmaceutical formulations which are useful with the dosing system of the invention include all pharmaceutically acceptable salts and conjugates thereof. Other topically-active compounds are listed in Remington's Pharmaceutical Sciences, 17th Ed., Merck Publishing Co., Easton, Pa. (1985), pages 773-791 and pages 1054-1058 (hereinafter Remington's).

The dosing system of the present invention can also be used for topical application of other preparations, such as for cosmetic purposes, e.g., antiperspirants, sunblocks, keratolitics, skin softeners, fragrances and anti-acne preparations.

These agents include sun screens such as p-dimethylaminobenzoic acid; skin softeners such as urea; keratolytic agents such as salicylic acid; acne agents such as benzoyl peroxide, perfumes and the like.

Suitable antiperspirant compositions include astringent salts. The astringent salts include organic and inorganic salts of aluminum, zirconium, zinc, and mixtures thereof. The anion of the astringent salt can be, for example, sulfate, chloride, chlorohydroxide, alum, formate, lactate, benzyl sulfonate or phenyl sulfonate. Exemplary classes of antiperspirant astringent salts include aluminum halides, aluminum hydroxyhalides, zirconyl oxyhalides, zirconyl hydroxyhalides, and mixtures thereof.

Exemplary aluminum salts include aluminum chloride and the aluminum hydroxyhalides. Exemplary zirconium compounds include zirconium oxy salts and zirconium hydroxy salts, also referred to as zirconyl salts and zirconyl hydroxy salts.

Exemplary antiperspirant compounds therefore include, but are not limited to, aluminum bromohydrate, potassium alum, sodium aluminum chlorohydroxy lactate, aluminum sulfate, aluminum chlorohydrate, aluminum-zirconium tetrachlorohydrate, an aluminum-zirconium polychlorohydrate complexed with glycine, aluminum-zirconium trichlorohydrate, aluminum-zirconium octachlorohydrate, aluminum sesquichlorohydrate, aluminum sesquichlorohydrex PG, aluminum chlorohydrex PEG, aluminum zirconium octachlorohydrex glycine complex, aluminum zirconium pentachlorohydrex glycine complex, aluminum zirconium tetrachlorohydrex glycine complex, aluminum zirconium trichlorohydrex glycine complex, aluminum chlorohydrex PG, zirconium chlorhydrate, aluminum dichlorohydrate, aluminum dichlorohydrex PEG, aluminum dichlorohydrex PG, aluminum sesquichlorohydrex PG, aluminum chloride, aluminum zirconium pentachlorohydrate, and mixtures thereof. Numerous other useful antiperspirant compounds are listed in WO 91/19222 and in the Cosmetic and Toiletry Fragrance Handbook, The Cosmetic, Toiletry and Fragrance Association, Inc., Washington, D.C., p. 56, 1989, hereinafter the CTFA Handbook.

Therapeutic agents having non-local activity for use with the dosing system of the invention include without limitation, active substances for use in the treatment or prevention of various systemic and disorders and their symptoms, such as disorders of the cardiovascular system, disorders of the muscles or joints, disorders of the organs. More particularly, therapeutic agents having non-local activity for use according to any aspect of the present invention include, for example, active substances for use as vasodilators, active substances for the treatment of motion sickness, contraceptive agents, hormone replacement agents, painkillers, smoking cessation aids, or any therapeutically effective combination thereof.

Advantageously, therapeutic agents having non-local activity for use according to any aspect of the present invention are selected from the group consisting of nitroglycerin, scopolamine, estradiol, norethisterone, fentanyl and nicotine, or any therapeutically effective combination thereof.

Pharmaceutical formulations useful with the dosing system of the present invention may include any suitable carrier for topical delivery. Suitable carriers include polymers such as sodium alginate, gelatin, corn starch, gum tragacanth, methylcellulose, hydroxyethylcellulose, carboxymethylcellulose, xanthan gum, dextrin, carboxymethylstarch, polyvinyl alcohol, sodium polyacrylate, methoxyethylene-maleic anhydride copolymer, polyvinyl ether, polyvinylpyrrolidone.

The carrier can be a cellulose, one or more glycerides (such as for example, one or more glycerol esters of saturated acids or one or more polyglycolysed glycerides, cocoa butter, theobroma or the like), one or more high molecular weight polyethylene glycol, one or more polyoxyethylene, lanolin and derivatives thereof, and one or more fatty acids, fatty alcohols, fatty acid esters (including, for example, caprylic acid, caprylic triglyceride or the like), any of which preceding ingredients can be optionally mixed with one or more organic oils (including, for example hydrogenated vegetable oils) or the like.

A carrier medium suitable for use in a pharmaceutical formulation useful with the dosing system of the invention can comprise a wax, a fat an oil or a combination thereof, including, without limitation, for example beeswax, olive oil, cocoa butter, sesame oil, soybean oil, camellia oil, peanut oil, beef fat, lard and lanolin.

In certain embodiments of the carrier medium comprises white petrolatum or a paraffin. In other embodiments the carrier medium includes a higher fatty acid, for example stearic acid. In certain embodiments the carrier comprises a higher alcohol, such as for example, cetyl alcohol, stearyl alcohol and combinations thereof. In other aspects of the invention, the carrier comprises the polyethylene glycol or water.

Any and all combinations of pharmaceutical excipients which provide a suitable vehicle for the drug when used in the present invention are meant to be encompassed by the present invention. Further excipients are know to those skilled in the art as described in Remington's Pharmaceutical Sciences, 17th Ed., Merck Publishing Co., Easton; Pa. (1985).

There is also described a method for topically administering a pharmaceutical formulation to a skin of a mammal, the method including: (i) actuating a dosing system comprising a dosing device including: a housing holding at least one unit dose of a pharmaceutical formulation comprising a drug and a carrier medium therefore; an applicator adapted for topically administering at least a unit dose of the pharmaceutical formulation directly onto the skin of the mammal; and an actuator, wherein upon actuation, the dosing device meters a unit dose of the pharmaceutical formulation from the housing to the applicator; and (ii) applying the unit dose directly onto the skin of the mammal with the applicator.

The method for topically administering a pharmaceutical formulation may further include re-actuating the dosing system and administering additional unit doses of the pharmaceutical formulation. The method is applicable for topically administering pharmaceutical formulations wherein the drug provides a local or a systemic effect.

In another aspect, the present invention relates to, in part, a method of preparing the dosing system for topical delivery of a pharmaceutical formulation including preparing at least one unit dose of a pharmaceutical preparation comprising a drug and a carrier; and placing the at least one unit dose into a dosing device comprising a housing for holding the at least one unit dose of a pharmaceutical; an applicator adapted for topically administering a unit dose of the pharmaceutical formulation directly onto the skin; and an actuator, wherein upon actuation, the device meters a unit dose of the pharmaceutical formulation from the housing to the applicator.

### DETAILED DESCRIPTION OF CERTAIN EMBODIMENTS

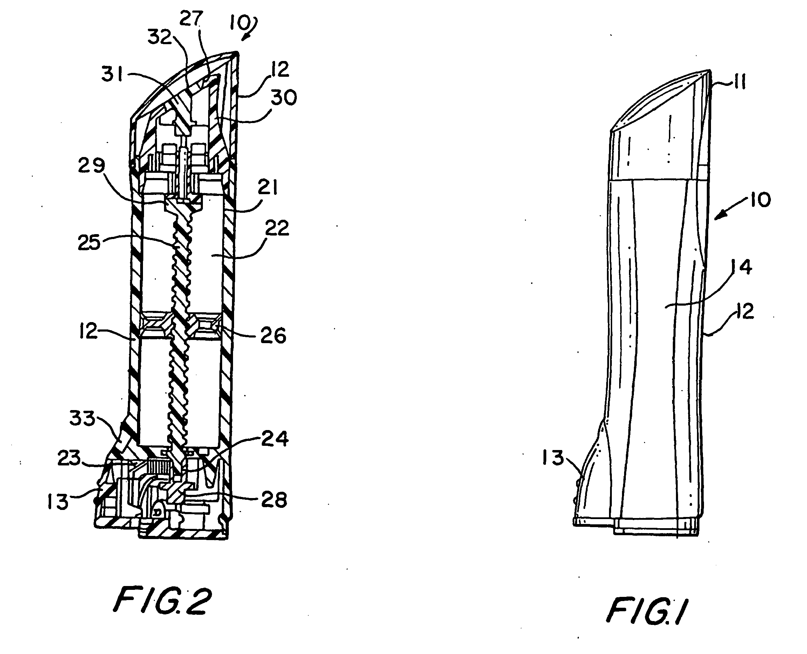

Fig. 1 shows the exterior of one embodiment of dosing device 10 with protective cap 11 shown in a closed position attached to an upper end of main body 12. Protective cap 11 may include side portions 14 to provide an attachment to main body 12 also in an opened position, which will be explained further below. Main body 12 may have a generally cylindrical shape and device 10 may be sized so as to be hand-held. Button 13 may be disposed at a lower end of main body 12.

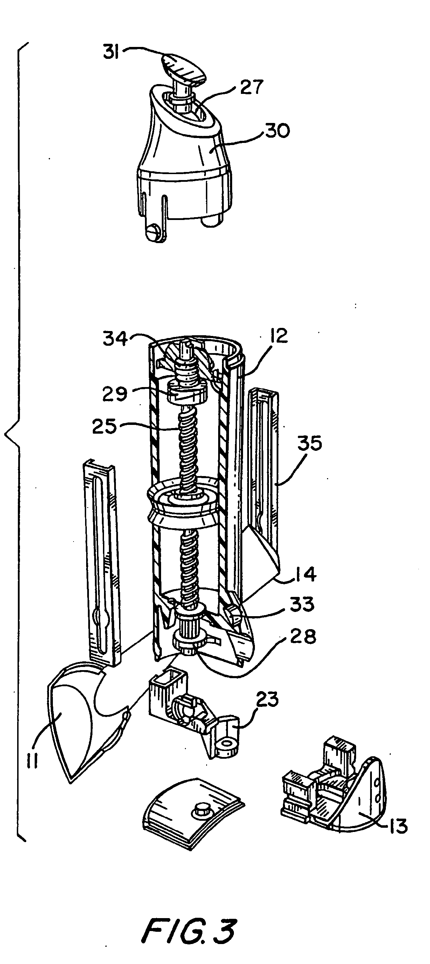

Referring to Figs. 2 and 3, pharmaceutical formulation 22, which may include a drug and a carrier, is held within cylindrical housing 21 of main body 12. Button 13 may be rigidly connected to rack 23, which is in operative connection with pinion 24, so that a displacement of rack 23 in its longitudinal direction causes pinion 24 to rotate. Pinion 24 may be rigidly connected to lead screw 25 which may be disposed longitudinally within housing 21. Lead screw 25 in turn may be operatively connected to piston 26 in such a way so that a rotation of lead screw 25 causes a displacement of piston 26 in a longitudinal direction of lead screw 25. Piston 26 may be disc-shaped and extend from lead screw 25 at its center to an inner wall of housing 21 at its outer perimeter. Preferably a seal is formed between piston 26 and both lead screw 25 and housing 25, so that upon actuation of button 13, rack 23 causes pinion 24 and lead screw 25 to rotate, thus causing piston 26 to move incrementally in an upward direction pushing pharmaceutical formulation 22 upward with it. Button 13, rack 23, pinion 24, lead screw 25, piston 26 and housing 21 may be configured, for example, so that each time button 13 is fully depressed, lead screw 25 rotates 120 degrees, thus causing piston 26 to move upward 1mm along lead screw 25 so as to discharge 0.5 grams of pharmaceutical compound 22 through outlet opening 27 of housing 21 and onto applicator head 30. Outlet valve 31 covers outlet opening 27 in its closed position. Outer surface 32 of outlet valve 31 forms part of applicator head in that it is used to apply pharmaceutical formulation 22 to the skin (not shown). Each successive actuation of button 13 causes a metered unit dose (for example 0.5 grams) of pharmaceutical formulation 22 to discharge from outlet opening 27 and onto applicator head 30. Dose counter window 33 may be disposed in main body 12 of device 10 to enable a user to read a display of a counter (not shown) in order to know how many unit doses of pharmaceutical formulation 22 have been discharged.

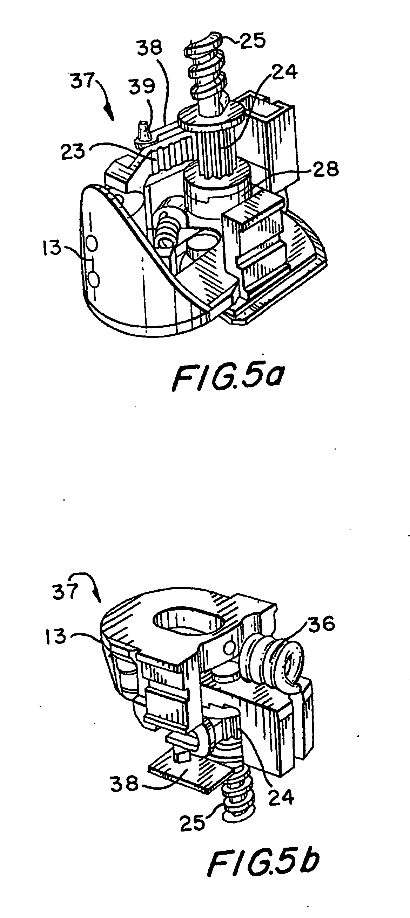

Lead screw 25, at its lower end, may be operatively connected to lower logic 28. Lower logic 28 may be configured to cause lead screw to displace in a longitudinally upward direction (for example by 1 mm) as button 23 is depressed, and to drop back down again to its original longitudinal position when button 23 reaches its fully depressed position or when button 23 travels back to its original (not actuated) position, (see also Fig. 5a). This action serves to prevent backpressure from building up within housing 21, which might otherwise cause device 10 to leak.

Fig. 3 shows a section view of protective cover 11 in an open position, i.e. not attached to the upper end of main body 12. Protective cover 11 may include side portions 14 in operative connection with side clamps 35 -- for example via pins of side portions 14 (not shown) and slots in side clamps 35 -- to provide a connection to main body 12 in both closed and opened positions. This feature serves to prevent protective cover 11 from being misplaced during use. Other features such as a tether, hinge, or other connection between protective cover 11 and main body 12 may be used instead to provide a similar function. In its closed position, protective cover 11 serves to protect applicator head 30 from contamination. Side portions 14 and side clamps 14 may be configured to enable protective cap 11 to attach to the lower end of main body 12 so as not to interfere with use of dosing device 10.

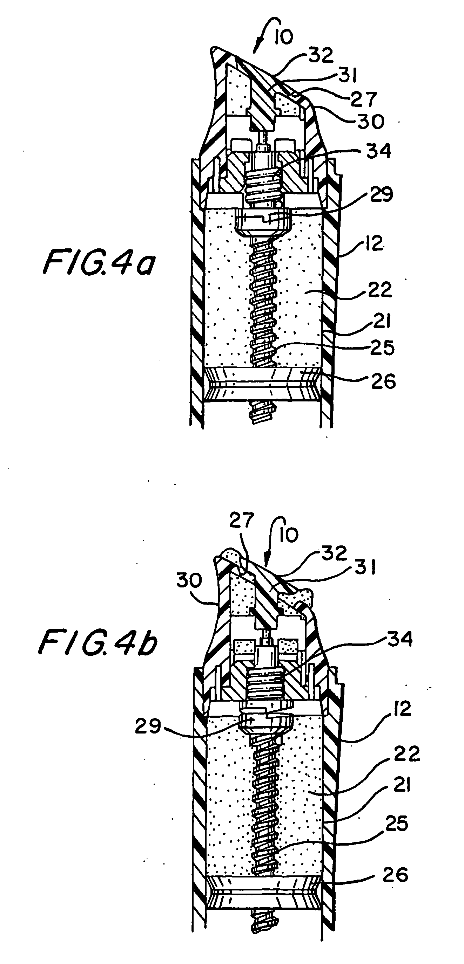

Lead screw 25, at its lower end, may be operatively connected with upper logic 29, which may include, for example, a pair of disk-shaped components having opposing ramps. In the example in which a complete depression of button 13 cause a 120 degree rotation of lead screw 25, the disk shape components may be configured to have three opposing ramps, each covering a 120 degree arc of the disk. Fig. 4a shows an upper portion of device 10 with outlet valve in a closed position and Fig. 4a shows the upper portion of device 10 with outlet valve 31 in an opened position. When lead screw 25 rotates, for example upon actuation of button 13, upper logic 29 may function to lift outlet valve 31 so that outlet valve 31 reaches its maximum height at the end of the incremental rotation movement of lead screw 25 (i.e. when button 13 is in its fully depressed position). For example, if lower logic 28 is configured to displace lead screw 25 in a longitudinal direction of 1mm during a single actuation of button 13, then upper logic 29 may be configured to displace outlet valve 31 in an upward direction by approximately 2 mm, in order to provide ample room in outlet opening 27 for discharge of pharmaceutical formulation 22. Outlet valve spring 34 may be disposed between outlet valve 31 and housing 21 to provide tension between the two components tending to move outlet valve 31 to a closed position. Thus, when lead screw 25 reaches the end of its incremental rotational movement, upper logic 29 and outlet valve spring 34 cause outlet valve 31 to move to its closed position. During the time that the valve is opened a metered unit dose of pharmaceutical formulation is discharged through outlet opening 27 and onto applicator head 30.

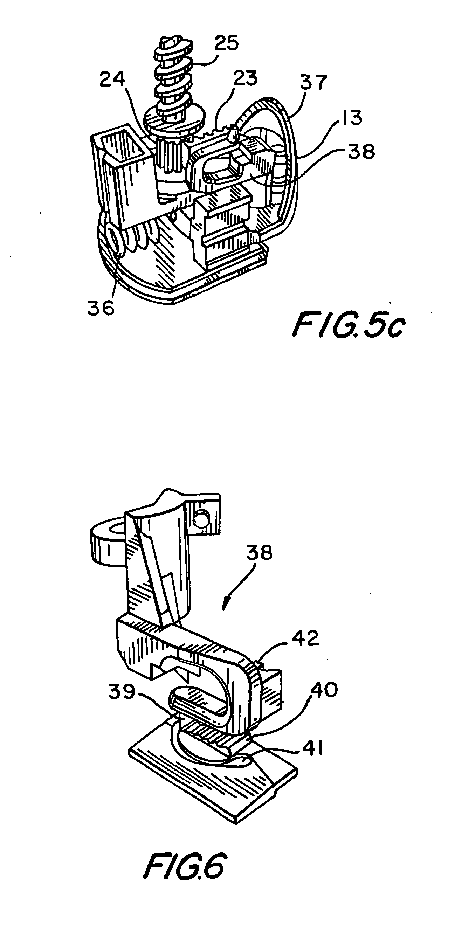

Figs. 5a, 5b, and 5c show perspective views of actuator 37 according to the present invention. As described above, actuation of button 13 may cause displacement of rack 23 to rotate pinion 24, thus rotating lead screw 25. Lower logic 28, comprising in this embodiment two opposing ramped disks cause lead screw 25 to displace in a longitudinally upward direction during one incremental rotational movement, and to drop back down to its original longitudinal position when button 13 is fully depressed or when button 13 returns to its non-depressed position. Button return spring 36 is disposed between button 13 and main body 22 so as to tend to move button 13 to its non-depressed position.

Fig. 6 shows a perspective view of one embodiment of a non-return mechanism that may be used with the dosing device 10 of Fig. 1. In a dosing device according to the present invention it is desirable to prevent a user from dispensing an amount of pharmaceutical formulation other than the predetermined metered unit dose. Therefore, it may be desirable to incorporate a non-return mechanism such as, for example, non-return mechanism 38 of Fig. 6, in order to prevent a user from partially actuating the actuator and thus dispensing less than the full unit dose upon actuation of the device.

Referring to Fig. 6, non-return mechanism 38 includes pin 39 in operative connection with ramp platform 40. Pin 39 may be rigidly connected to button 13 (not shown in Fig. 6) and ramp platform 40 may be rigidly connected to main body 12 (not shown in Fig. 6). Likewise, pin 39 may be rigidly connected to main body 12 with ramp rack 40 rigidly connected to button 13. Pin compression spring 42 provides a force tending to keep pin 39 in contact with ramp platform 40. As button 13 is depressed pin 39 moves along ramp platform 40, and is able to ride up over each of the ramps on ramp platform 40 in one direction, but is prevented by a ratchet effect from traveling over the ramps in the opposite direction. Thus, if button 13 is not fully depressed, anti-return mechanism 38 prevents button 13 from returning to its non-depressed position because pin 39 can only travel in one direction along ramp rack 40. Ramp rack 40 may have a length corresponding to a travel distance of button 13 so that when button 13 reaches its fully depressed position, pin 39 reaches the end of ramp rack 40 and falls into return track 41. Track 41 guides pin 39 around ramp rack 40 as button 13 travels back to its non-depressed position (via force provided by button return spring 36 (not shown in Fig. 6) so that pin 39 is returned to its starting position at the beginning end of ramp rack 40.

The present invention has been described herein with reference to specific exemplary embodiments thereof. It will, however, be evident that various modifications and changes may be made thereto without departing from the scope of the invention as set forth in the claims that follow. The specification and drawings are accordingly to be regarded in an illustrative manner rather than a restrictive sense. A person of skill in the art will, of course, appreciate many ways of implementing the present invention, in addition to the embodiments described in Figs. 1-6. For example, many well-known structures can be used instead of using a piston and lead screw to create pressure on the pharmaceutical formulation within the housing. For instance, if the housing were a flexible tube, a roller or pair of rollers could be used to squeeze the tube and cause the formulation to be discharged. The push may be replaced by a sliding button, lever, or rotary knob. An actuator could control movement of the rollers to cause a metered unit dose of the formulation to be discharged. The formulation within the housing may also, for example, be pre-pressurized using a propellant and the actuator could cause metered unit dosages to be discharged using by actuating a metering valve.

## Claims

1. A dosing device (10) for topically administering a pharmaceutical formulation (22) to the skin of a mammal, the device comprising: a housing (21) capable of storing at least one dose of a pharmaceutical formulation (22) comprising a drug incorporated with a pharmaceutically acceptable carrier suitable for topical application onto the skin of said mammal; an applicator (30) adapted for topically administering a dose of the pharmaceutical formulation (22) directly onto the skin; and an actuator (13) capable of metering a single dose of the pharmaceutical formulation (22) from a first position in which the dose is stored in the housing (21) to a second position in which the single dose is external to the device on the applicator (30) so that the single dose can be topically administered,
**characterised in that** the device is a unitary device which includes a valve (31) disposed in an opening of the housing (21), wherein the valve (31) is moveable between an open position to allow discharge of the dose through the opening to the applicator (30) and a closed position to seal the opening, and wherein air is substantially prevented from entering the housing (21) during or after actuation.

2. A dosing device (10) as claimed in claim 1, wherein said housing (21) is capable of holding multiple doses of the pharmaceutical formulation (22).

3. A dosing device (10) as claimed in claim 1, wherein said housing (21) is:
(a) adapted to hold a semisolid pharmaceutical formulation (22), preferably a semisolid pharmaceutical formulation selected from an ointment, gel, emulsion, lotion, spray, cream and paste; or
(b) adapted to hold a liquid pharmaceutical formulation, preferably a liquid pharmaceutical formulation selected from a suspension and a solution.

4. A dosing device (10) as claimed in claim 1, wherein upon actuation, the device is capable of metering a dose of pharmaceutical formulation (22) from about 0.10 grams to about 5 grams from the housing (21) to the applicator (30), preferably wherein the device is capable of metering a dose of pharmaceutical formulation (22) of about 1.0 gram from the housing (21) to the applicator (30).

5. A dosing device as claimed in claim 1, wherein upon a subsequent actuation, an additional dose is capable of being delivered from the housing (21) to the applicator (30), until depletion of the pharmaceutical formulation (22) from the housing (21).

6. A dosing device (10) as claimed in claim 5, further comprising a means for preventing the actuator (13) from functioning after a predetermined number of actuations, for a predetermined time period, wherein the means is preferably a mechanical means or an electrical means.

7. A dosing device (10) as claimed in claim 6, wherein the desired time period is the dosing interval of the drug, preferably wherein the dosing interval is from about 1 hour to about 24 hours, or from about 4 hour to about 12 hours.

8. A dosing device (10) as claimed in claim 6, wherein the predetermined number of actuation is a number which administers the prescribed amount of pharmaceutical formulation (22), preferably wherein the predetermined number of actuations is one actuation or more than one actuation.

9. A dosing device (10) as claimed in claim 6, wherein the desired time period is at least the time needed to topically administer the previously metered dose, in order to prevent accidental actuation during application.

10. A dosing device (10) as claimed in claim 1, wherein each dose metered from the device does not vary:
(a) by more than about 10% at room temperature;
(b) by more than about 10% at a temperature from about 20°C to about 40°C;
(c) by more than about 10% at a temperature from about 10°C to about 80°C;
(d) by more than about 10% at a temperature from greater than 0°C to less than about 100°C;
(e) by more than about 5% at room temperature;
(f) by more than about 5% at a temperature from about 20°C to about 40°C;
(g) by more than about 5% at a temperature from about 10°C to about 80°C;
(h) by more than about 5% at a temperature from greater than 0°C to less than about 100°C;
(i) by more than about 1% at room temperature;
(j) by more than about 1 % at a temperature from about 20°C to about 40°C;
(k) by more than about 1% at a temperature from about 10°C to about 80°C; or
(l) by more than about 1% at a temperature from greater than 0°C to less than 100°C.

11. A dosing device (10) as claimed in claim 1, wherein said applicator (30) comprises a flat surface, preferably wherein said flat surface is angled from a baseline perpendicular to said device (10) when said device (10) is held upright.

12. A dosing device (10) as claimed in claim 1, wherein said applicator (30) comprises a convex surface.

13. A dosing device (10) as claimed in claim 1, wherein said applicator (30) is comprised of a material which inhibits microbial proliferation, preferably wherein said material comprises a silver containing plastic

14. A dosing device (10) as claimed in either of claims 1 and 12, wherein said housing (21) is comprised of a material which inhibits microbial proliferation, preferably wherein said material comprises a silver containing plastic.

15. A dosing device (10) as claimed in claim 1, wherein said applicator (30) is comprised of a non-wetting material which promotes the formation of droplets when exposed to moisture, preferably wherein said material comprises silicone.

16. A dosing device (10) as claimed in either of claims 1 and 14, wherein said housing (21) is comprised of a non-wetting material which promotes the formation of droplets when exposed to moisture, preferably wherein said material comprises silicone.

17. A dosing device (10) as claimed in either of claims 11 and 12, wherein said applicator surface is smooth or ridged.

18. A dosing device (10) as claimed in claim 1, further comprising a counter which indicates:
(a) the number of doses remaining in the system; or
(b) the number of doses actuated,
preferably wherein the counter is mechanical or electronic.

19. A dosing device (10) as claimed in claim 1, wherein after depletion or partial depletion of said at least one dose, said device (10) is capable of being reloaded with at least one additional dose.

20. A dosing device (10) as claimed in claim 1, wherein after depletion of said at least one dose, said device (10) is incapable of being reloaded and is disposable.

21. A dosing device (10) as claimed in claim 2, wherein said housing (21) is adapted to contain :
(a) at least 5 doses of said pharmaceutical formulation (22);
(b) from about 5 doses to about 400 doses of said pharmaceutical formulation (22);
(c) from about 40 doses to about 120 doses of said pharmaceutical formulation (22);
(d) from about 30 doses to about 100 doses of said pharmaceutical formulation (22);
(e) about 365 doses of said pharmaceutical formulation (22); or
(f) about 30 doses of said pharmaceutical formulation (22).

22. A dosing device (10) as claimed in claim 1, wherein actuation of the actuator (13) causes a positive pressure in the housing (21), the positive pressure causing the valve (31) to move from the closed position to the open position.

23. A dosing device (10) as claimed in claim 1, wherein said actuator (13) comprises a button wherein actuation of the button causes a dose to be discharged from the housing (21) to the applicator (30).

24. A dosing device (10) as claimed in claim 1, wherein the actuator (13) comprises a button, a rack, a pinion, and a lead screw (25) in operative connection with each other, and wherein actuation of the button causes the dose to be discharged from the housing (21) to the applicator (30).

25. A dosing device (10) as claimed in claim 23, further comprising a protective cover (11) adapted to cover the valve (31) and applicator (30) in a closed position.

26. A dosing device (10) as claimed in claim 23, wherein said valve (31) is movable from the open position to the closed position by a spring mechanism.

27. A dosing device (10) as claimed in claim 23, wherein the button is moveable between a non-actuated position and an actuated position, preferably wherein the device (10) further comprises a button spring mechanism which causes the button to return to the non-actuated position after actuation.

28. A dosing device (10) as claimed in claim 1, further comprising a non-return mechanism adapted to prevent the actuator (13) from delivering a partial dose and/or contaminating the device (10).

29. A dosing device (10) as claimed in claim 24 wherein the lead screw (25):
(a) includes a ratchet logic adapted to reduce a back pressure in the container; or
(b) further comprises a valve logic for moving the valve from the closed position to the open position.

30. A dosing device (10) as claimed in claim 1, wherein the applicator (30):
(a) includes a roller ball adapted to roll the dose onto the skin; or
(b) comprises a static surface rigidly connected with the housing (21) and adapted to spread the dose onto the skin.

31. A dosing device (10) as claimed in claim 1, wherein the actuator (13) is flush with the surface of the device (10), or wherein the actuator (13) is recessed from the surface of the device (10).

32. A dosing device (10) as claimed in claim 1, which can be submersed in water for at least 30 seconds without having the housing (21) infiltrated with water.

33. A dosing device (10) as claimed in claim 1, wherein the housing (21) is:
(a) airtight;
(b) comprised of aluminium at the point of contact with the composition; or
(c) comprised of plastic coated with aluminium at the point of contact with the composition.

34. A dosing device (10) as claimed in claim 1, comprising a visual aid to determine the number of doses remaining in the device (10), preferably wherein said visual aid is a transparent window to the inside of the housing (21).

35. A dosing device (10) as claimed in claim 1, which allows less than about 10% overage of the pharmaceutical composition (22) or less than about 5% overage of the pharmaceutical composition (22).

36. A dosing device (10) as claimed in claim 1, which does not require overage of the pharmaceutical composition (22).

37. A dosing device (10) as claimed in any of claims 1-36, further comprising a pharmaceutical formulation (22) comprising a drug and a carrier, the pharmaceutical formulation (22) being suitable for topical application.

38. A dosing device (10) as claimed in claim 37, wherein said formulation is a semisolid, preferably wherein said semisolid is selected from an ointment, gel, emulsion, lotion, spray, cream and paste.

39. A dosing device (10) as claimed in claim 37, wherein the formulation is a liquid, preferably wherein said liquid is:
(a) a suspension or a solution; or
(b) converted to a foam upon actuation and metering of a dose from said housing (21) to said applicator (30).

40. A dosing device (10) as claimed in claim 37, wherein at least about 95% of said drug is absorbed by the skin over a period of less than about 30 minutes after administration, preferably over a period of less than about 20 minutes after administration or over a period of less than about 5 minutes after administration.

41. A dosing device (10) as claimed in claim 37, wherein not more than about 50% of said drug is absorbed by the skin over a period of about 12 hours or more after administration, preferably over a period of about 6 hours or more after administration or over a period of about 2 hours or more after administration.

42. A dosing device (10) as claimed in claim 37, wherein said drug is selected from ACE inhibitors, adenohypophoseal hormones, adrenergic neuron blocking agents, adrenocortical steroids, inhibitors of the biosynthesis of adrenocortical steroids, alpha-adrenergic agonists, alpha-adrenergic antagonists, selective alpha₂-adrenergic agonists, analgesics, antipyretics, anti-inflammatory agents, androgens, local anesthetics, general anesthetics, antiaddictive agents, antiandrogens, antiarrhythmic agents, antiasthmatic agents, anticholinergic agents, anticholinesterase agents, anticoagulants, antidiabetic agents, antidiarrheal agents, antidiuretic agents, antiemetic agents, prokinetic agents, antiepileptic agents, antiestrogens, antifungal agents, antihypertensive agents, antimicrobial agents, antimigraine agents, antimuscarinic agents, antineoplastic agents, antiparasitic agents, antiparkinson agents, antiplatelet agents, antiprogestins, antithyroid agents, antitussives, antiviral agents, antidepressants, azaspirodecanediones, barbituates, benzodiazepines, benzothiadiazides, beta-adrenergic agonists, beta-adrenergic antagonists, selective beta₁-adrenergic antagonists, selective beta₂-adtenergic agonists, bile salts, agents affecting volume and composition of body fluids, butyrophenones, agents affecting calcification, calcium channel blockers, cardiovascular drugs, catecholamines, sympathomimetic drugs, cholinergic agonists, cholinesterase reactivators, dermatological agents, diphenylbutylpiperidines, diuretics, ergot alkaloids, estrogens, ganglionic blocking agents, ganglionic stimulating agents, hydantoins, agents for control of gastric acidity, agents for treatment of peptic ulcers, hematopoietic agents, anti-histamines, 5-hydroxytryptamine antagonists, drugs for the treatment of hyperlipoproteinemia, hypnotics, sedatives, immunosupressive agents, laxatives, bronchodilators, monoamine oxidase inhibitors, neuromuscular blocking agents, organic nitrates, pancreatic enzymes, phenothiazines, progestins, prostaglandins, anti-psychotic agents, retinoids, sodium channel blockers, agents for spasticity, agents for acute muscle spasms, succinimides, xanthines, thrombolytic agents, thyroid agents, tricyclic antidepressants, inhibitors of tubular transport of organic compounds, drugs affecting uterine motility, vasodilators and vitamins, bepridil, diltiazem, felodipine, isradipine, nicardipine, nifedipine, nimodipine, nitredipine, verapamil, dobutamine, isoproterenol, carterolol, labetalol, levobunolol, nadolol, penbutolol, pindolol, propranolol, sotalol, timolol, acebutolol, atenolol, betaxolol, esmolol, metoprolol, albuterol, bitolterol, isoetharine, metaproterenol, pirbuterol, ritodrine, terbutaline, alclometasone, aldosterone, amcinonide, beclomethasone, dipropionate, betamethasone, clobetasol, clocortolone, cortisol, cortisone, corticosterone, desonide, desoximetasone, 11-desoxycorticosterone, 11-desoxycortisol, dexamethasone, diflorasone, fludrocortisone, flunisolide, fluocinolone, fluocinonide, fluorometholone, flurandrenolide, halcinonide, hydrocortisone, medrysone, 6.alpha.-methylprednisolone, mometasone, paramethasone, prednisolone, prednisone, tetrahydrocortisol, triamcinolone, benoxinate, benzocaine, bupivacaine, chloroprocaine, cocaine, dibucaine, dyclonine, etidocaine, lidocaine, mepivacaine, pramoxine, prilocaine, procaine, proparacaine, tetracaine, alfentanil, choroform, clonidine, cyclopropane, desflurane, diethyl ether, droperidol, enflurane, etomidate, halothane, isoflurane, ketamine hydrochloride, meperidine, methohexital, methoxyflurane, morphine, propofol, sevoflurane, thiamylal, thiopental, acetaminophen, allopurinol, apazone, aspirin, auranofin, aurothioglucose, colchicine, diclofenac, diflunisal, etodolac, fenoprofen, flurbiprofen, gold sodium thiomalate, ibuprofen, indomethacin, ketoprofen, meclofenamate, mefenamic acid, meselamine, methyl salicylate, nabumetone, naproxen, oxyphenbutazone, phenacetin, phenylbutazone, piroxicam, salicylamide, salicylate, salicylic acid, salsalate, sulfasalazine, sulindac, tolmetin, acetophenazine, chlorpromazine, fluphenazine, mesoridazine, perphenazine, thioridazine, trifluorperazine, triflupromazine, disopyramide, encainide, flecainide, indecainide, mexiletine, moricizine, phenytoin, procainamide, propafenone, quinidine, tocainide, cisapride, domperidone, dronabinol, haloperidol, metoclopramide, nabilone, prochlorperazine, promethazine, thiethylperazine, trimethobenzamide, buprenorphine, butorphanol, dezocine, diphenoxylate, drocode, hydrocodone, hydromorphone, levallorphan, levorphanol, loperamide, meptazinol, methadone, nalbuphine, nalmefene, nalorphine, naloxone, naltrexone, oxybutynin, pentazocine, isosorbide dinitrate, nitroglycerin, theophylline, phenylephrine, ephidrine, pilocarpine, furosemide, tetracycline, chlorpheniramine, ketorolac, bromocriptine, guanabenz, prazosin, doxazosin, flufenamic acid and pharmaceutically acceptable salts thereof, benzodiazepines, such as alprazolam, brotizolam, chlordiazepoxide, clobazam, clonazepam, clorazepate, demoxepam, diazepam, flumazenil, flurazepam, halazepam, lorazepam, midazolam, nitrazepam, nordazepam, oxazepam, prazepam, quazepam, temazepam, triazolam, and the like; an antimuscarinic agent such as anisotropine, atropine, clidinium, cyclopentolate, dicyclomine, flavoxate, glycopyrrolate, hexocyclium, homatropine, ipratropium, isopropamide, mepenzolate, methantheline, oxyphencyclimine, pirenzepine, propantheline, scopolamine, telenzepine, tridihexethyl, tropicamide, and the like; an estrogen such as chlorotrianisene, siethylstilbestrol, methyl estradiol, estrone, estrone sodium sulfate, estropipate, mestranol, quinestrol, sodium equilin sulfate, 17.beta.-estradiol (or estradiol), semi-synthetic estrogen derivatives such as the esters of natural estrogen, such as estradiol-17.beta.-enanthate, estradiol-17.beta.-valerate, estradiol-3-benzoate, estradiol-17.beta.-undecenoate, estradiol 16,17-hemisuccinate or estradiol-17.beta.-cypionate, and the 17-alkylated estrogens, such as ethinyl estradiol, ethinyl estradiol-3-isopropylsulphonate, and the like; an androgen such as danazol, fluoxymesterone, methandrostenolone, methyltestosterone, nandrolone decanoate, nandrolone phenpropionate, oxandrolone, oxymetholone, stanozolol, testolactone, testosterone, testosterone cypionate, testosterone enanthate, testosterone propionate, and the like; or a progestin such as ethynodiol diacetate, gestodene, hydroxyprogesterone caproate, levonorgestrel, medroxyprogesterone acetate, megestrol acetate, norethindrone, norethindrone acetate, norethynodrel, norgestrel, progesterone, and pharmaceutically acceptable salts thereof.

43. A dosing device (10) as claimed in claim 37, wherein said drug is a locally active agent.

44. A dosing device (10)as claimed in claim 43, wherein the locally active agent is for use in the treatment of disorders of the skin selected from the group consisting of psoriasis, eczema, acne, nappy rash, other inflammatory disorders, bacterial infections, viral infections, fungal infections, anaphylactic conditions, malignancies and warts.

45. A dosing device (10) as claimed in claim 43, wherein the locally active agent is selected from the group consisting of anesthetics, corticosteroids, antibacterial agents, antifungal agents or any therapeutically effective combination thereof. Preferably wherein said anesthetic is selected from bupivacaine, levo-bupivacaine, ropivacaine, benzocaine, dibucaine, procaine, chloroprocaine, prilocaine, mepivacaine, etidocaine, tetracaine, lidocaine, and xylocaine, as well as anesthetically active derivatives, analogs, isomers and mixtures thereof.

46. A dosing device (10) as claimed in claim 43, wherein the locally active agent is selected from tetracaine, benzocaine, lindocaine, hydrocortisone, beclomethasone diproprionate, clobetasol proprionate, fluticasone proprionate, ichthammol, lithium succinate, coal tar, dithranol, benzoyl peroxide, tretinoin, sulphur, vitamin D and derivatives thereof, framycetin, chlortetracycline hydrochloride, fusidic acid, clotrimazole, econazole, amorolfine and terbenafine, or any therapeutically effective combination thereof.

47. A dosing device (10) as claimed in claim 43, wherein the locally active agent is selected from antiviral agents (e.g., acyclovir and idoxuridine, etc.), antifungal agents (e.g., amphotericin B, clotrimazole, nystatin, ketoconazole, miconazole, butocouazole, haloprogin, etc.), antibiotic agents (penicillins, cephalosporins erythromycin, tetracycline, clindamycin, aminoglycosides, chloramphenicol, polymixin b, bacitracin, neomycin, gentamycin, etc.), antiseptics (e.g., povidone-iodine, methylbenzethonium chloride, etc.), antiparasitics (e.g., lindane, anthralin, etc.) analgesic agents (e.g., methylsalicylate, salicylic acid, dyclonine, aloe vera, etc.), local anesthetics (e.g., benzocaine, lidocaine, xylocaine, butamben picrate, etc.), anti-inflammatory agents (e.g., steroidal compounds such as dexamethasone, betamethasone, prednisone, prednisolone, triamcinolone, hydrocortisone, alclometasone, amcinonide, diflorasone, etc., as well as non-steroidal anti-inflammatories), anti-itch and irritation-reducing compounds (e.g., antihistamines such as diphenhydramine and psoriasis treatments); burn relief compounds (e.g., o-amino-p-toluenesulfonamide, monoacetate, etc.); depigmenting agents (e.g., monobenzone); and hormonal agents (e.g., oestriol).

48. A dosing device (10) as claimed in claim 37, wherein said drug has non-local activity.

49. A dosing device (10) as claimed in claim 48, wherein said drug is selected from vasodilators, active substances for the treatment of motion sickness, contraceptive agents, hormone replacement agents, painkillers, and smoking cessation aids, or any therapeutically effective combination thereof, nitroglycerin, scopolamine, estradiol, norethisterone, fentanyl and nicotine, or any therapeutically effective combination thereof.

50. A dosing device (10) as claimed in claim 37, wherein said carrier comprises a polymer, preferably wherein said polymer is selected from sodium alginate, gelatin, corn starch, gum tragacanth, methylcellulose, hydroxyethylcellulose, carboxymethylcellulose, xanthan gum, dextrin, carboxymethylstarch, polyvinyl alcohol, sodium polyacrylate, methoxyethylene-maleic anhydride copolymer, polyvinyl ether, polyvinylpyrrolidone.

51. A dosing device (10) as claimed in claim 37, wherein said carrier comprises a wax a fat, an oil or a combination thereof, preferably wherein said fat or oil is selected from beeswax, olive oil, cacao butter, sesame oil, soybean oil, camellia oil, peanut oil, beef fat, lard and lanolin.

52. A dosing device (10) as claimed in claim 37, wherein said carrier comprises:
(a) white petrolatum;
(b) a paraffin;
(c) a higher fatty acid, preferably stearic acid;
(d) a higher alcohol, preferably selected from cetyl alcohol, stearyl alcohol and combinations thereof;
(e) a polyethylene glycol; or
(f) water.

53. A dosing device (10) as claimed in claim 37, wherein said drug comprises nitrogen oxide.

54. A dosing device (10) as claimed in any of claims 1-52, for topically administering a pharmaceutical formulation (22) to the skin of a mammal, wherein the device (10) is actuated and the dose applied directly onto the skin of a mammal with the applicator (30).

55. A dosing device (10) as claimed in claim 54, wherein the device may be actuated a second time and additional doses of said pharmaceutical formulation (22) administered.

56. A dosing device (10) as claimed in claim 54, wherein said drug:
(a) provides a local effect on the surface of the skin;
(b) is absorbed and provides a local effect in the region of application; or
(c) is absorbed and provides a systemic effect.

57. A method of preparing a dosing system for topical delivery of a pharmaceutical formulation (22) comprising:
(i) preparing at least one dose of a pharmaceutical preparation comprising a drug incorporated with a pharmaceutically acceptable carrier suitable for topical application onto the skin of said mammal; and
(ii) placing the at least one dose into a dosing device according to claim 1.

## Patentansprüche

1. Dosiervorrichtung (10) zur topischen Verabreichung einer pharmazeutischen Zusammensetzung (22) auf die Haut eines Säugers, wobei die Vorrichtung Folgendes umfasst: ein Gehäuse (21), das mindestens eine Dosis einer pharmazeutischen Zusammensetzung (22) lagern kann, die ein mit einem pharmazeutisch verträglichen Träger integriertes Arzneimittel umfasst, der sich zum topischen Aufbringen auf die Haut des Säugers eignet; eine Auftragsvorrichtung (30), die zur topischen Verabreichung einer Dosis der pharmazeutischen Zusammensetzung (22) direkt auf die Haut ausgeführt ist; und eine Betätigungsvorrichtung (13), die eine Einzeldosis der pharmazeutischen Zusammensetzung (22) aus einer ersten Position, in der die Dosis im Gehäuse (21) gelagert ist, in eine zweite Position, in der sich die Einzeldosis außerhalb der Vorrichtung auf der Auftragsvorrichtung (30) befindet, zumessen kann, so dass die Einzeldosis topisch verabreicht werden kann,
**dadurch gekennzeichnet, dass** die Vorrichtung eine einteilige Vorrichtung ist, die ein Ventil (31) enthält, das in einer Öffnung des Gehäuses (21) angeordnet ist, wobei das Ventil (31) zwischen einer geöffneten Position, um ein Abführen der Dosis durch die Öffnung zu der Auftragsvorrichtung (30) zu gestatten, und einer geschlossenen Position zum Abdichten der Öffnung, beweglich ist, und im Wesentlichen verhindert wird, dass Luft während oder nach der Betätigung in das Gehäuse (21) eintritt.

2. Dosiervorrichtung (10) nach Anspruch 1, wobei das Gehäuse (21) mehrere Dosen der pharmazeutischen Zusammensetzung (22) aufnehmen kann.

3. Dosiervorrichtung (10) nach Anspruch 1, wobei das Gehäuse (21) :
(a) zur Aufnahme einer halbfesten pharmazeutischen Zusammensetzung (22), vorzugsweise einer halbfesten pharmazeutischen Zusammensetzung, die unter einer Salbe, einem Gel, einer Emulsion, einer Lotion, einem Spray, einer Creme und einer Paste ausgewählt ist, ausgeführt ist, oder
(b) zur Aufnahme einer flüssigen pharmazeutischen Zusammensetzung, vorzugsweise einer flüssigen pharmazeutischen Zusammensetzung, die unter einer Suspension und einer Lösung ausgewählt ist, ausgeführt ist.

4. Dosiervorrichtung (10) nach Anspruch 1, wobei die Vorrichtung bei Betätigung eine Dosis der pharmazeutischen Zusammensetzung (22) von ca. 0,10 g bis ca. 5 g aus dem Gehäuse (21) zur Auftragsvorrichtung (30) zumessen kann, vorzugsweise wobei die Vorrichtung eine Dosis der pharmazeutischen Zusammensetzung (22) von ca. 1,0 g aus dem Gehäuse (21) zu der Auftragsvorrichtung (30) zumessen kann.

5. Dosiervorrichtung nach Anspruch 1, wobei bei anschließender Betätigung eine zusätzliche Dosis aus dem Gehäuse (21) der Auftragsvorrichtung (30) zugeführt werden kann, bis die pharmazeutische Zusammensetzung (22) aus dem Gehäuse (21) aufgebraucht worden ist.

6. Dosiervorrichtung (10) nach Anspruch 5, die weiterhin ein Mittel zum Verhindern für eine vorbestimmte Zeitdauer, dass die Betätigungsvorrichtung (13) nach einer vorbestimmten Anzahl von Betätigungen funktioniert, umfasst, wobei das Mittel vorzugsweise ein mechanisches Mittel oder ein elektrisches Mittel ist.

7. Dosiervorrichtung (10) nach Anspruch 6, wobei die gewünschte Zeitdauer das Dosierintervall des Arzneimittels ist, wobei das Dosierintervall vorzugsweise von ca. 1 Stunde bis ca. 24 Stunden oder von ca. 4 Stunden bis ca. 12 Stunden reicht.

8. Dosiervorrichtung (10) nach Anspruch 6, wobei die vorbestimmte Anzahl von Betätigungen eine Anzahl ist, die die vorgeschriebene Menge an pharmazeutischer Zusammensetzung (22) verabreicht, wobei die vorbestimmte Anzahl von Betätigungen vorzugsweise eine Betätigung oder mehr als eine Betätigung ist.

9. Dosiervorrichtung (10) nach Anspruch 6, wobei die gewünschte Zeitdauer mindestens die Zeit ist, die dazu erforderlich ist, die zuvor zugemessene Dosis zu verabreichen, um eine versehentliche Betätigung während des Auftragens zu verhindern.

10. Dosiervorrichtung (10) nach Anspruch 1, wobei keine der von der Vorrichtung zugemessenen Dosen:
(a) um mehr als ca. 10% bei Raumtemperatur,
(b) um mehr als ca. 10% bei einer Temperatur von ca. 20°C bis ca. 40°C,
(c) um mehr als ca. 10% bei einer Temperatur von ca. 10°C bis ca. 80°C,
(d) um mehr als ca. 10% bei einer Temperatur von über 0°C bis weniger als ca. 100°C,
(e) um mehr als ca. 5% bei Raumtemperatur;
(f) um mehr als ca. 5% bei einer Temperatur von ca. 20°C bis ca. 40°C,
(g) um mehr als ca. 5% bei einer Temperatur von ca. 10°C bis ca. 80°C;
(h) um mehr als ca. 5% bei einer Temperatur von über 0°C bis weniger als ca. 100°C;
(i) um mehr als ca. 1% bei Raumtemperatur;
(j) um mehr als ca. 1% bei einer Temperatur von ca. 20°C bis ca. 40°C
(k) um mehr als ca. 1% bei einer Temperatur von ca. 10°C bis ca. 80°C oder
(l) um mehr als ca. 1% bei einer Temperatur von über 0°C bis weniger als 100°C
variiert.

11. Dosiervorrichtung (10) nach Anspruch 1, wobei die Auftragsvorrichtung (30) eine flache Fläche umfasst, wobei die flache Fläche vorzugsweise von einer senkrecht zu der Vorrichtung (10) verlaufenden Grundlinie abgewinkelt ist, wenn die Vorrichtung (10) aufrecht gehalten wird.

12. Dosiervorrichtung (10) nach Anspruch 1, wobei die Auftragsvorrichtung (30) eine konvexe Fläche umfasst.

13. Dosiervorrichtung (10) nach Anspruch 1, wobei die Auftragsvorrichtung (30) aus einem Material besteht, das eine mikrobielle Proliferation verhindert, vorzugsweise wobei das Material einen silberhaltigen Kunststoff umfasst.

14. Dosiervorrichtung (10) nach einem der Ansprüche 1 und 12, wobei das Gehäuse (21) aus einem Material besteht, das eine mikrobielle Proliferation verhindert, vorzugsweise wobei das Material einen silberhaltigen Kunststoff umfasst.

15. Dosiervorrichtung (10) nach Anspruch 1, wobei die Auftragsvorrichtung (30) aus einem nicht benetzenden Material besteht, das die Bildung von Tröpfchen fördert, wenn es Feuchtigkeit ausgesetzt ist, vorzugsweise wobei das Material Silikon umfasst.

16. Dosiervorrichtung (10) nach einem der Ansprüche 1 und 14, wobei das Gehäuse (21) aus einem nicht benetzenden Material besteht, das die Bildung von Tröpfchen fördert, wenn es Feuchtigkeit ausgesetzt ist, vorzugsweise wobei das Material Silikon umfasst.

17. Dosiervorrichtung (10) nach einem der Ansprüche 11 und 12, wobei die Auftragsvorrichtungsfläche glatt oder geriffelt ist.

18. Dosiervorrichtung (10) nach Anspruch 1, die weiterhin einen Zähler umfasst, der:
(a) die Anzahl der im System verbleibenden Dosen oder
(b) die Anzahl der betätigten Dosen anzeigt, vorzugsweise wobei der Zähler mechanisch oder elektronisch ist.

19. Dosiervorrichtung (10) nach Anspruch 1, wobei die Vorrichtung (10) nach dem Aufbrauchen oder teilweisen Aufbrauchen der mindestens einen Dosis mit mindestens einer zusätzlichen Dosis nachgeladen werden kann.

20. Dosiervorrichtung (10) nach Anspruch 1, wobei die Vorrichtung (10) nach dem Aufbrauchen der mindestens einen Dosis nicht nachgeladen werden kann und eine Wegwerf-Vorrichtung ist.

21. Dosiervorrichtung (10) nach Anspruch 2, wobei das Gehäuse (21) dazu ausgeführt ist:
(a) mindestens 5 Dosen der pharmazeutischen Zusammensetzung (22),
(b) von ca. 5 Dosen bis ca. 400 Dosen der pharmazeutischen Zusammensetzung (22),
(c) von ca. 40 Dosen bis ca. 120 Dosen der pharmazeutischen Zusammensetzung (22),
(d) von ca. 30 Dosen bis ca. 100 Dosen der pharmazeutischen Zusammensetzung (22),
(e) ca. 365 Dosen der pharmazeutischen Zusammensetzung (22) oder
(f) ca. 30 Dosen der pharmazeutischen Zusammensetzung (22)
aufzunehmen.

22. Dosiervorrichtung (10) nach Anspruch 1, wobei eine Betätigung der Betätigungsvorrichtung (13) einen Überdruck in dem Gehäuse (21) verursacht, wobei der Überdruck bewirkt, dass sich das Ventil (31) aus der geschlossenen Position in die geöffnete Position bewegt.

23. Dosiervorrichtung (10) nach Anspruch 1, wobei die Betätigungsvorrichtung (13) einen Knopf umfasst, wobei eine Betätigung des Knopfes bewirkt, dass eine Dosis aus dem Gehäuse (21) zur Auftragsvorrichtung (30) abgeführt wird.

24. Dosiervorrichtung (10) nach Anspruch 1, wobei die Betätigungsvorrichtung (13) einen Knopf, eine Zahnstange, ein Ritzel und eine Gewindespindel (25) umfasst, die in Wirkverbindung miteinander stehen, und wobei eine Betätigung des Knopfes bewirkt, dass die Dosis aus dem Gehäuse (21) zur Auftragsvorrichtung (30) abgeführt wird.

25. Dosiervorrichtung (10) nach Anspruch 23, die weiterhin eine Schutzabdeckung (11) umfasst, um das Ventil (31) und die Auftragsvorrichtung (30) in einer geschlossenen Position abzudecken.

26. Dosiervorrichtung (10) nach Anspruch 23, wobei das Ventil (31) durch einen Federmechanismus aus der geöffneten Position in die geschlossene Position beweglich ist.

27. Dosiervorrichtung (10) nach Anspruch 23, wobei der Knopf zwischen einer nicht betätigten Position und einer betätigten Position beweglich ist, vorzugsweise wobei die Vorrichtung (10) weiterhin einen Knopffedermechanismus umfasst, der bewirkt, dass der Knopf nach der Betätigung in die nicht betätigte Position zurückkehrt.

28. Dosiervorrichtung (10) nach Anspruch 1, die weiterhin einen Nichtrückstellmechanismus umfasst, der dazu ausgeführt ist zu verhindern, dass die Betätigungsvorrichtung (13) eine Teildosis abgibt und/oder die Vorrichtung (10) verunreinigt.

29. Dosiervorrichtung (10) nach Anspruch 24, wobei die Gewindespindel (25):
(a) eine Sperrklinkenlogik enthält, die zur Verringerung des Gegendrucks im Behälter ausgeführt ist, oder
(b) weiterhin eine Ventillogik zum Bewegen des Ventils aus der geschlossenen Position in die geöffnete Position enthält.

30. Dosiervorrichtung (10) nach Anspruch 1, wobei die Auftragsvorrichtung (30):
(a) eine Rollkugel enthält, die dazu ausgeführt ist, die Dosis auf die Haut zu rollen, oder
(b) eine statische Fläche umfasst, die starr mit dem Gehäuse (21) verbunden ist und dazu ausgeführt ist, die Dosis auf die Haut aufzustreichen,

31. Dosiervorrichtung (10) nach Anspruch 1, wobei die Betätigungsvorrichtung (13) bündig mit der Fläche der Vorrichtung (10) ausgebildet ist oder wobei die Betätigungsvorrichtung (13) von der Oberfläche der Vorrichtung (10) zurückgesetzt ist.

32. Dosiervorrichtung (10) nach Anspruch 1, die für mindestens 30 Sekunden in Wasser eingetaucht werden kann, ohne dass Wasser in das Gehäuse (21) eindringt.

33. Dosiervorrichtung (10) nach Anspruch 1, wobei das Gehäuse (21) :
(a) luftdicht ist,
(b) an der Berührungsstelle mit der Zusammensetzung aus Aluminium besteht oder
(c) an der Berührungsstelle mit der Zusammensetzung aus mit Aluminium beschichtetem Kunststoff besteht.

34. Dosiervorrichtung (10) nach Anspruch 1, die eine optische Hilfe zur Bestimmung der in der Vorrichtung (10) verbleibenden Anzahl von Dosen umfasst, vorzugsweise wobei die optische Hilfe ein durchsichtiges Fenster in das Innere des Gehäuses (21) ist.

35. Dosiervorrichtung (10) nach Anspruch 1, die weniger als ca. 10% Zuschlag der pharmazeutischen Zusammensetzung (22) oder weniger als ca. 5% Zuschlag der pharmazeutischen Zusammensetzung (22) gestattet.

36. Dosiervorrichtung (10) nach Anspruch 1, die keinen Zuschlag der pharmazeutischen Zusammensetzung (22) erfordert.

37. Dosiervorrichtung (10) nach einem der Ansprüche 1 - 36, die weiterhin eine pharmazeutische Zusammensetzung (22) umfasst, die ein Arzneimittel und einen Träger umfasst, wobei die pharmazeutische Zusammensetzung (22) zur topischen Verabreichung geeignet ist.

38. Dosiervorrichtung (10) nach Anspruch 37, wobei die Zusammensetzung eine halbfeste Substanz ist, vorzugsweise wobei die halbfeste Substanz unter einer Salbe, einem Gel, einer Emulsion, einer Lotion, einem Spray, einer Creme und einer Paste ausgewählt ist.

39. Dosiervorrichtung (10) nach Anspruch 37, wobei die Zusammensetzung eine Flüssigkeit ist, vorzugsweise wobei die Flüssigkeit:
(a) eine Suspension oder eine Lösung ist; oder
(b) bei Betätigung und Zumessung einer Dosis aus dem Gehäuse (21) zur Auftragsvorrichtung (30) in einen Schaum umgewandelt wird.

40. Dosiervorrichtung (10) nach Anspruch 37, wobei mindestens ca. 95% des Arzneimittels über einen Zeitraum von ca. 30 Minuten nach Verabreichung, vorzugsweise über einen Zeitraum von weniger als ca. 20 Minuten nach Verabreichung oder über einen Zeitraum von weniger als ca. 5 Minuten nach Verabreichung von der Haut absorbiert wird.

41. Dosiervorrichtung (10) nach Anspruch 37, wobei nicht mehr als ca. 50% des Arzneimittels über einen Zeitraum von ca. 12 Stunden oder mehr nach Verabreichung, vorzugsweise über einen Zeitraum von ca. 6 Stunden oder mehr nach Verabreichung oder über einen Zeitraum von ca. 2 Stunden oder mehr nach Verabreichung von der Haut absorbiert wird.

42. Vorrichtung (10) nach Anspruch 37, wobei das Arzneimittel ausgewählt ist aus ACE-Hemmern, Adenohypophysenhormonen, adrenergen Neuronenblockern, adrenocorticalen Steroiden, Inhibitoren der Biosynthese adrenocorticaler Steroide, alpha-adrenergen Agonisten, alpha-adrenergen Antagonisten, selektiven alpha₂-adrenergen Agonisten, Analgetika, Antipyretika, entzündungshemmenden Mitteln, Androgenen, Lokalanästhetika, allgemeinen Anästhetika, Antisuchtmitteln, Antiandrogenen, Antiarrhythmika, Antiasthmatika, Anticholinergika, Anticholinesterase-Mitteln, Antikoagulantien, Antidiabetika, Antidiarrhoe-Mitteln, Antidiuretika, Antiemetika, prokinetischen Mitteln, Antiepileptika, Antiöstrogenen, pilzhemmenden Mitteln, Antihypertensiva, antimikrobiellen Mitteln, Antimigränemitteln, Antimuskarinmitteln, antineoplastischen Mitteln, parasitenhemmenden Mitteln, Antiparkinson-Mitteln, Thrombozytenaggregationshemmern, Antiprogestinen, Thyreostatika, Antitussiva, antiviralen Mitteln, Antidepressiva, Azaspirodecandionen, Barbituraten, Benzodiazepinen, Benzothiadiaziden, beta-adrenergen Agonisten, beta-adrenergen Antagonisten, selektiven beta₁-adrenergen Antagonisten, selektiven beta₂-adrenergen Agonisten, Gallensalzen, Mitteln, die das Volumen und die Zusammensetzung von Körperflüssigkeiten beeinflussen, Butyrophenonen, Mitteln, die die Kalzifizierung beeinflussen, Calciumkanalblockern, kardiovaskulären Arzneimitteln, Catecholaminen, Sympathomimetika, cholinergen Agonisten, Cholinesterasereaktivatoren, dermatologischen Mitteln, Diphenylbutylpiperidinen, Diuretika, Ergotalkaloiden, Östrogenen, Ganglionblockern, Ganglionstimulatoren, Hydantoinen, Mitteln zur Steuerung der Magenazidität, Mitteln zur Behandlung von Magengeschwüren, hämatopoetischen Mitteln, Antihistaminika, 5-Hydroxytryptamin-Antagonisten, Arzneimitteln zur Behandlung von Hyperlipoproteinämie, Hypnotika, Sedativa, Immunsupressiva, Laxativa, Bronchodilatatoren, Monoaminoxidase-Hemmern, neuromuskulären Blockern, organischen Nitraten, Pankreasenzymen, Phenothiazinen, Progestinen, Prostaglandinen, Antipsychosemitteln, Retinoiden, Natriumkanalblockern, Mitteln gegen spastische Lähmung, Mitteln gegen akute Muskelspasmen, Succinimiden, Xanthinen, Thrombolysemitteln, Thyroidmitteln, tricyclischen Antidepressiva, Inhibitoren des tubulären Transports organischer Verbindungen, Arzneimitteln, die die Uterusmotilität beeinflussen, Vasodilatatoren und Vitaminen, Bepridil, Diltiazem, Felodipin, Isradipin, Nicardipin, Nifedipin, Nimodipin, Nitredipin, Verapamil, Dobutamin, Isoproterenol, Carterolol, Labetalol, Levobunolol, Nadolol, Penbutolol, Pindolol, Propranolol, Sotalol, Timolol, Acebutolol, Atenolol, Betaxolol, Esmolol, Metoprolol, Albuterol, Bitolterol, Isoetharin, Metaproterenol, Pirbuterol, Ritodrin, Terbutalin, Alclometason, Aldosteron, Amcinonid, Beclomethason, Dipropionat, Betamethason, Clobetasol, Clocortolon, Cortisol, Cortison, Corticosteron, Desonid, Desoximetason, 11-Desoxycorticosteron, 11-Desoxycortisol, Dexamethason, Diflorason, Fludrocortison, Flunisolid, Fluocinolon, Fluocinonid, Fluorometholon, Flurandrenolid, Halcinonid, Hydrocortison, Medryson, 6. alpha.-Methylprednisolon, Mometason, Paramethason, Prednisolon, Prednison, Tetrahydrocortisol, Triamcinolon, Benoxinat, Benzocain, Bupivacain, Chloroprocain, Cocain, Dibucain, Dyclonin, Etidocain, Lidocain, Mepivacain, Pramoxin, Prilocain, Procain, Proparacain, Tetracain, Alfentanil, Chloroform, Clonidin, Cyclopropan, Desfluran, Diethylether, Droperidol, Enfluran, Etomidat, Halothan, Isofluran, Ketaminhydrochlorid, Meperidin, Methohexital, Methoxyfluran, Morphin, Propofol, Sevofluran, Thiamylal, Thiopental, Acetaminophen, Allopurinol, Apazon, Aspirin, Auranofin, Aurothioglucose, Colchicin, Diclofenac, Diflunisal, Etodolac, Fenoprofen, Flurbiprofen, Gold-Natriumthiomalat, Ibuprofen, Indomethacin, Ketoprofen, Meclofenamat, Mefenaminsäure, Meselamin, Methylsalicylat, Nabumeton, Naproxen, Oxyphenbutazon, Phenacetin, Phenylbutazon, Piroxicam, Salicylamid, Salicylat, Salicylsäure, Salsalat, Sulfasalazin, Sulindac, Tolmetin, Acetophenazin, Chlorpromazin, Fluphenazin, Mesoridazin, Perphenazin, Thioridazin, Trifluorperazin, Triflupromazin, Disopyramid, Encainid, Flecainid, Indecainid, Mexiletin, Moricizin, Phenytoin, Procainamid, Propafenon, Chinidin, Tocainid, Cisaprid, Domperidon, Dronabinol, Haloperidol, Metoclopramid, Nabilon, Prochlorperazin, Promethazin, Thiethylperazin, Trimethobenzamid, Buprenorphin, Butorphanol, Dezocin, Diphenoxylat, Drocod, Hydrocodon, Hydromorphon, Levallorphan, Levorphanol, Loperamid, Meptazinol, Methadon, Nalbuphin, Nalmefen, Nalorphin, Naloxon, Naltrexon, Oxybutynin, Pentazocin, Isosorbiddinitrat, Nitroglycerin, Theophyllin, Phenylephrin, Ephidrin, Pilocarpin, Furosemid, Tetracyclin, Chlorpheniramin, Ketorolac, Bromocriptin, Guanabenz, Prazosin, Doxazosin, Flufenaminsäure und pharmazeutisch verträglichen Salzen davon, Benzodiazepinen, wie Alprazolam, Brotizolam, Chlordiazepoxid, Clobazam, Clonazepam, Clorazepat, Demoxepam, Diazepam, Flumazenil, Flurazepam, Halazepam, Lorazepam, Midazolam, Nitrazepam, Nordazepam, Oxazepam, Prazepam, Quazepam, Temazepam, Triazolam, und dergleichen; einem Antimuskarinmittel wie Anisotropin, Atropin, Clidinium, Cyclopentolat, Dicyclomin, Flavoxat, Glykopyrrolat, Hexocyclium, Homatropin, Ipratropium, Isopropamid, Mepenzolat, Methanthelin, Oxyphencyclimin, Pirenzepin, Propanthelin, Scopolamin, Telenzepin, Tridihexethyl, Tropicamid, und dergleichen; einem Östrogen wie Chlorotrianisen, Siethylstilbestrol, Methylöstradiol, Östron, Östronnatriumsulfat, Östropipat, Mestranol, Chinestrol, Natriumequilinsulfat, 17.beta.-Östradiol (oder Östradiol), semi-synthetischen Östrogen-Derivativen, wie die Ester von natürlichem Östrogen, wie Östradiol 17. beta . -enanthat, Östradiol-17.beta.-valerat, Östradiol-3-benzoat, Östradiol-17.beta.-undecenoat, Östradiol-16, 17-hemisuccinat oder Östradiol-17.beta.-cypionat, und den 1 7-alkylierten Östrogenen, wie Ethinylöstradiol, Ethinylöstradiol-3-isopropylsulfonat, und dergleichen; einem Androgen, wie Danazol, Fluoxymesteron, Methandrostenolon, Methyltestosteron, Nandrolondecanoat, Nandrolonphenpropionat, Oxandrolon, Oxymetholon, Stanozolol, Testolacton, Testosteron, Testosteroncypionat, Testosteronenanthat, Testosteronpropionat, und dergleichen; oder einem Progestin, wie Ethynodioldiacetat, Gestoden , Hydroxyprogesteroncaproat, Levonorgestrel, Medroxyprogesteronacetat, Megestrolacetat, Norethindron, Norethindronacetat, Norethynodrel, Norgestrel, Progesteron, und pharmazeutisch verträglichen Salzen davon.

43. Dosiervorrichtung (10) nach Anspruch 37, wobei das Arzneimittel ein lokal aktives Mittel ist.

44. Dosiervorrichtung (10) nach Anspruch 43, wobei das lokal aktive Mittel zur Behandlung von Störungen der Haut, ausgewählt aus der Gruppe, bestehend aus Psoriasis, Ekzem, Akne, Windelausschlag, anderen entzündlichen Störungen, Bakterieninfektionen, Virusinfektionen, Pilzinfektionen, anaphylaktischen Zuständen, Malignitäten und Warzen, verwendet wird.

45. Dosiervorrichtung (10) nach Anspruch 43, wobei das lokal aktive Mittel ausgewählt ist aus der Gruppe, bestehend aus Anästhetika, Corticosteroiden, Antibakterienmitteln, Antipilzmitteln, oder einer beliebigen therapeutisch wirksamen Kombination davon, wobei das Anästhetikum vorzugsweise ausgewählt ist aus Bupivacain, Levobupivacain, Ropivacain, Benzocain, Dibucain, Procain, Chlorprocain, Prilocain, Mepivacain, Etidocain, Tetracain, Lidocain und Xylocain, sowie anästhetisch aktiven Derivaten, Analoga, Isomeren und Gemischen davon.

46. Dosiervorrichtung (10) nach Anspruch 43, wobei das lokal aktive Mittel ausgewählt ist aus Tetracain, Benzocain, Lindocain, Hydrocortison, Beclomethasondipropionat, Clobetasolproprionat, Fluticasonproprionat, Ichthammol, Lithiumsuccinat, Steinkohleteer, Dithranol, Benzoylperoxid, Tretinoin, Schwefel, Vitamin D und Derivaten davon, Framycetin, Chlortetracyclinhydrochlorid, Fusidinsäure, Clotrimazol, Econazol, Amorolfin und Terbenafin oder einer beliebigen therapeutisch wirksamen Kombination davon.

47. Dosiervorrichtung nach Anspruch 43, wobei das lokal aktive Mittel ausgewählt ist aus Antivirusmitteln (zum Beispiel Acyclovir und Idoxuridin usw.), Antipilzmitteln (zum Beispiel Amphotericin B, Clotrimazol, Nystatin, Ketoconazol, Miconazol, Butocouazol, Haloprogin usw.), Antibiotika (Penicillinen, Cephalosporinen, Erythromycin, Tetracyclin, Clindamycin, Aminoglykosiden, Chloramphenicol, Polymixin b, Bacitracin, Neomycin, Gentamycin usw.), Antiseptika (zum Beispiel Povidoniod, Methylbenzethoniumchlorid usw.), Antiparasitika (zum Beispiel Lindan, Anthralin usw.) Analgetika (zum Beispiel Methylsalicylat, Salicylsäure, Dyclonin, Aloe vera usw.), Lokalanästhetika (zum Beispiel Benzocain, Lidocain, Xylocain, Butambenpicrat usw.), entzündungshemmenden Mitteln (zum Beispiel Steroid-Verbindungen, wie Dexamethason, Betamethason, Prednison, Prednisolon, Triamcinolon, Hydrocortison, Alclometason, Amcinonid, Diflorason usw., sowie nicht-steroidalen Entzündungshemmern), juckreizunterdrückenden and entzündungslindernden Verbindungen (zum Beispiel Antihistaminika, wie Diphenhydramin und Psoriasis-Behandlungen); verbrennungslindernde Verbindungen (zum Beispiel o-Amino-p-toluolsulfonamid, Monoacetat usw.); Depigmentierungsmitteln (zum Beispiel Monobenzon); und Hormonmitteln (zum Beispiel Östriol).

48. Dosiervorrichtung (10) nach Anspruch 37, wobei das Arzneimittel nicht-lokale Aktivität aufweist.

49. Dosiervorrichtung (10) nach Anspruch 48, wobei das Arzneimittel ausgewählt ist aus Vasodilatatoren, aktiven Substanzen zur Behandlung von Bewegungskrankheit, Kontrazeptiva, Hormonersatzmitteln, schmerzstillenden Mitteln und Mitteln, die es einem erleichtern, mit dem Rauchen aufzuhören, oder jeder therapeutisch wirksamen Kombination davon, Nitroglycerin, Scopolamin, Östradiol, Norethisteron, Fentanyl und Nikotin, oder jeder beliebigen Kombination davon.

50. Dosiervorrichtung (10) nach Anspruch 37, wobei der Träger ein Polymer umfasst, wobei das Polymer vorzugsweise ausgewählt ist aus Natriumalginat, Gelatine, Maisstärke, Tragantgummi, Methylcellulose, Hydroxyethylcellulose, Carboxymethylcellulose, Xanthangummi, Dextrin, Carboxymethylstärke, Polyvinylalkohol, Natriumpolyacrylat, Methoxyethylen-Maleinsäureanhydrid-Copolymer, Polyvinylether, Polyvinylpyrrolidon.

51. Dosiervorrichtung (10) nach Anspruch 37, wobei der Träger ein Wachs, ein Fett, ein Öl oder eine Kombination davon umfasst, wobei das Fett oder Öl vorzugsweise ausgewählt ist aus Bienenwachs, Olivenöl, Kakaobutter, Sesamöl, Sojaöl, Kamelienöl, Erdnussöl, Rinderfett, Schmalz und Lanolin.

52. Dosiervorrichtung (10) nach Anspruch 37, wobei der Träger umfasst:
(a) weiße Vaseline,
(b) ein Paraffin,
(c) eine höhere Fettsäure, vorzugsweise Stearinsäure,
(d) einen höheren Alkohol, vorzugsweise ausgewählt aus Cetylalkohol, Stearylalkohol und Kombinationen davon,
(e) ein Polyethylenglykol oder
(f) Wasser.

53. Dosiervorrichtung (10) nach Anspruch 37, wobei das Arzneimittel Stickstoffoxid umfasst.

54. Dosiervorrichtung (10) nach einem der Ansprüche 1 - 52 zur topischen Verabreichung einer pharmazeutischen Formulierung (22) auf die Haut eines Säugers, wobei die Vorrichtung (10) betätigt wird und die Dosis mit der Auftragsvorrichtung (30) direkt auf die Haut eines Säugetiers aufgetragen wird.

55. Dosiervorrichtung (10) nach Anspruch 54, wobei die Vorrichtung ein zweites Mal betätigt werden kann und zusätzliche Dosen der pharmazeutischen Formulierung (22) verabreicht werden können.

56. Dosiervorrichtung (10) nach Anspruch 54, wobei das Arzneimittel:
(a) eine lokale Wirkung auf der Oberfläche der Haut hervorruft,
(b) absorbiert wird und eine lokale Wirkung im Auftragungsbereich hervorruft oder
(c) absorbiert wird und eine systemische Wirkung hervorruft.

57. Verfahren zur Herstellung eines Dosiersystems zur topischen Abgabe einer pharmazeutischen Formulierung (22), umfassend:
(i) Herstellen von mindestens einer Dosis eines pharmazeutischen Präparats, ein mit einem pharmazeutisch verträglichen Träger integriertes Arzneimittel umfasst, der sich zum topischen Aufbringen auf die Haut des Säugers eignet, und
(ii) Unterbringen der mindestens einen Dosis in einer Dosiervorrichtung nach Anspruch 1.

## Revendications

1. Dispositif de dosage (10) pour l'administration topique d'une formulation pharmaceutique (22) sur la peau d'un mammifère, le dispositif comprenant :
un logement (21) dans lequel on peut conserver au moins une dose d'une formulation pharmaceutique (22) contenant un médicament incorporé dans un excipient pharmaceutiquement acceptable qui convient pour une application topique sur la peau dudit mammifère,
un applicateur (30) conçu pour administrer de façon topique une dose de la formulation pharmaceutique (22) directement sur la peau et
un actionneur (13) qui permet de délivrer une dose unique de la formulation pharmaceutique (22) entre une première position dans laquelle la dose est conservée dans le logement (21) et une deuxième position dans laquelle la dose unique est placée à l'extérieur du dispositif sur l'applicateur (30) pour que la dose unique puisse être administrée de façon topique,
**caractérisé en ce que**
le dispositif est un dispositif intégré qui comprend une soupape (31) placée dans une ouverture du logement (21),
**en ce que** la soupape (31) est mobile entre une position ouverte qui permet le déversement de la dose par l'ouverture vers l'applicateur (30) et une position fermée qui permet de fermer de manière étanche l'ouverture et
**en ce que** on empêche essentiellement l'air d'entrer dans le logement (21) pendant ou après l'actionnement.

2. Dispositif de dosage (10) selon la revendication 1, dans lequel ledit logement (21) peut contenir plusieurs doses de la formulation pharmaceutique (22).

3. Dispositif de dosage (10) selon la revendication 1, dans lequel ledit logement (21) est :
(a) conçu pour contenir une formulation pharmaceutique semi-solide (22), de préférence une formulation pharmaceutique semi-solide sélectionnée dans l'ensemble constitué d'une pommade, d'un gel, d'une émulsion, d'une lotion, d'un spray, d'une crème et d'une pâte ou
(b) conçu pour contenir une formulation pharmaceutique liquide, de préférence une formulation pharmaceutique liquide sélectionnée dans l'ensemble constitué d'une suspension et d'une solution.

4. Dispositif de dosage (10) selon la revendication 1, dans lequel lors de l'actionnement, le dispositif peut mesurer une dose de la formulation pharmaceutique (22) allant du logement (21) vers l'applicateur (30), la dose étant comprise entre environ 0,10 grammes et environ 5 grammes, le dispositif étant de préférence capable de mesurer une dose d'environ 1,0 grammes de la formulation pharmaceutique (22) allant du logement (21) vers l'applicateur (30).

5. Dispositif de dosage selon la revendication 1, dans lequel lors d'un actionnement ultérieur, une dose supplémentaire peut être délivrée du logement (21) de l'applicateur (30) jusqu'à épuisement de la formulation pharmaceutique (22) contenue dans le logement (21).

6. Dispositif de dosage (10) selon la revendication 5, comprenant de plus des moyens qui empêchent l'actionneur (13) de fonctionner pendant une durée prédéterminée après un nombre prédéterminé d'actionnements, les moyens étant de préférence des moyens mécaniques ou des moyens électriques.

7. Dispositif de dosage (10) selon la revendication 6, dans lequel la durée souhaitée est l'intervalle de dosage du médicament, l'intervalle de dosage étant de préférence compris entre environ 1 heure et environ 24 heures ou entre environ 4 heures et environ 12 heures.

8. Dispositif de dosage (10) selon la revendication 6, dans lequel le nombre prédéterminé d'actionnements est un nombre qui permet d'administrer la quantité prescrite de la formulation pharmaceutique (22), le nombre prédéterminé d'actionnements valant de préférence au moins un.

9. Dispositif de dosage (10) selon la revendication 6, dans lequel la durée souhaitée est au moins la durée nécessaire pour administrer de façon topique la dose mesurée antérieurement, de manière à empêcher un actionnement accidentel pendant l'application.

10. Dispositif de dosage (10) selon la revendication 1, dans lequel chaque dose mesurée par le dispositif ne varie pas :
(a) de plus d'environ 10 % à température ambiante,
(b) de plus d'environ 10 % à une température comprise entre environ 20°C et environ 40°C,
(c) de plus d'environ 10 % à une température comprise entre environ 10°C et environ 80°C,
(d) de plus d'environ 10 % à une température comprise entre plus de 0°C à moins d'environ 100°C,
(e) de plus d'environ 5 % à température ambiante,
(f) de plus d'environ 5 % à une température comprise entre environ 20°C et environ 40°C,
(g) de plus d'environ 5 % à une température comprise entre environ 10°C à environ 80°C,
(h) de plus d'environ 5 % à une température supérieure à 0°C et inférieure à environ 100°C,
(i) de plus d'environ 1 % à température ambiante
(j) de plus d'environ 1 % à une température comprise entre environ 20°C à environ 40°C,
(k) de plus d'environ 1 % à une température comprise entre environ 10°C et environ 80°C, ou
(l) de plus d'environ 1 % à une température comprise entre plus de à 0°C et moins de 100°C.

11. Dispositif de dosage (10) selon la revendication 1, dans lequel ledit applicateur (30) comprend une surface plate, ladite surface plate étant de préférence oblique par rapport à une ligne de base perpendiculaire audit dispositif (10) lorsque ledit dispositif (10) est maintenu à la verticale.

12. Dispositif de dosage (10) selon la revendication 1, dans lequel ledit applicateur (30) comprend une surface convexe.

13. Dispositif de dosage (10) selon la revendication 1, dans lequel ledit applicateur (30) est constitué d'un matériau qui inhibe la prolifération microbienne, ledit matériau comprenant de préférence un plastique qui contient de l'argent.

14. Dispositif de dosage (10) selon la revendication 1 ou la revendication 12, dans lequel ledit logement (21) est constitué d'un matériau qui inhibe la prolifération microbienne, ledit matériau comprenant de préférence un plastique qui contient de l'argent.

15. Dispositif de dosage (10) selon la revendication 1, dans lequel ledit applicateur (30) est constitué d'un matériau non mouillable qui facilite la formation de gouttes lorsqu'il est exposé à l'humidité, ledit matériau comprenant de préférence du silicone.

16. Dispositif de dosage (10) selon l'une quelconque des revendications 1 à 14, dans lequel ledit logement (21) est constitué d'un matériau non mouillable qui favorise la formation de gouttes lorsqu'il est exposé à l'humidité, ledit matériau comprenant de préférence du silicone.

17. Dispositif de dosage (10) soit selon la revendication 11, soit selon la revendication 12, dans lequel ladite surface de l'applicateur est lisse ou striée.

18. Dispositif de dosage (10) selon la revendication 1, comprenant de plus un compteur qui indique :
(a) le nombre de doses qui reste dans le système ou
(b) le nombre de doses qui ont été délivrées, le compteur étant de préférence un compteur mécanique ou un compteur électronique.

19. Dispositif de dosage (10) selon la revendication 1, dans lequel après épuisement ou après épuisement partiel de ladite ou desdites doses, ledit dispositif (10) peut être rechargé d'au moins une dose supplémentaire.

20. Dispositif de dosage (10) selon la revendication 1, dans lequel après épuisement de ladite ou desdites doses, ledit dispositif (10) ne peut pas être rechargé et est jeté.

21. Dispositif de dosage (10) selon la revendication 2, dans lequel ledit logement (21) est conçu pour contenir :
(a) au moins 5 doses de ladite formulation pharmaceutique (22),
(b) d'environ 5 doses à environ 400 doses de ladite formulation pharmaceutique (22),
(c) d'environ 40 doses à environ 120 doses de ladite formulation pharmaceutique (22),
(d) d'environ 30 doses à environ 100 doses de ladite formulation pharmaceutique (22),
(e) environ 365 doses de ladite formulation pharmaceutique (22), ou
(f) environ 30 doses de ladite formulation pharmaceutique (22).

22. Dispositif de dosag e (10) selon la revendication 1, dans lequel l'actionnement de l'actionneur (13) induit une pression positive dans le logement (21), la pression positive amenant la soupape (31) à se déplacer de la position fermée vers la position ouverte.

23. Dispositif de dosage (10) selon la revendication 1, dans lequel ledit actionneur (13) comprend un bouton, l'actionnement du bouton induisant le déversement d'une dose du logement (21) vers l'applicateur (30).

24. Dispositif de dosage (10) selon la revendication 1, dans lequel l'actionneur (13) comprend un bouton, une crémaillère, un pignon et une tige filetée (25) qui en fonctionnement sont raccordés les uns aux autres, l'actionnement du bouton amenant le déversement de la dose du logement (21) vers l'applicateur (30).

25. Dispositif de dosage (10) selon la revendication 23, comprenant de plus un revêtement de protection (11) conçu pour couvrir en position fermée la soupape (31) et l'applicateur (30).

26. Dispositif de dosage (10) selon la revendication 23, dans lequel ledit clapet (31) est mobile de la position ouverte vers la position fermée grâce à un mécanisme à ressort.

27. Dispositif de dosage (10) selon la revendication 23, dans lequel le bouton est mobile entre une position non actionnée et une position actionnée, le dispositif (10) comprenant de préférence de plus un mécanisme à bouton à ressort qui amène le bouton à retourner vers la position non actionnée après l'actionnement.

28. Dispositif de dosage (10) selon la revendication 1, comprenant de plus un mécanisme de non-retour conçu pour empêcher l'actionneur (13) de délivrer une dose partielle et/ou de contaminer le dispositif (10).

29. Dispositif de dosage (10) selon la revendication 24, dans lequel la tige filetée 25) :
(a) comprend une logique d'encliquetage conçue pour réduire le reflux dans le récipient, ou
(b) comprend de plus une logique de soupape pour déplacer la soupape de la position fermée vers la position ouverte.

30. Dispositif de dosage (10) selon la revendication 1, dans lequel l'applicateur (30) :
(a) comprend une bille conçue pour faire rouler la dose sur la peau, ou
(b) comprend une surface statique reliée de façon rigide au logement (21) et conçue pour étaler la dose sur la peau.

31. Dispositif de dosage (10) selon la revendication 1, dans lequel l'actionneur (13) est au même niveau que la surface du dispositif (10) ou dans lequel l'actionneur (13) est creusé dans la surface du dispositif (10).

32. Dispositif de dosage (10) selon la revendication 1, qui peut être immergé dans de l'eau pendant au moins 30 secondes sans que de l'eau s'infiltre dans le logement (21).

33. Dispositif de dosage (10) selon la revendication 1, dans lequel le logement (21) est :
(a) étanche à l'air,
(b) contient de l'aluminium au point de contact avec la composition, ou
(c) contient un plastique recouvert d'aluminium au point de contact avec la composition.

34. Dispositif de dosage (10) selon la revendication 1, comprenant une aide visuelle pour déterminer le nombre de doses qui restent dans le dispositif (10), ladite aide visuelle étant de préférence une fenêtre transparente permettant de voir l'intérieur du logement (21).

35. Dispositif de dosage (10) selon la revendication 1, qui permet un surdosage de la composition pharmaceutique (22) inférieur à environ 10 % ou un surdosage de la composition pharmaceutique (22) inférieur à environ 5 %.

36. Dispositif de dosage (10) selon la revendication 1, qui ne requiert pas un surdosage de la composition pharmaceutique (22).

37. Dispositif de dosage (10) selon l'une quelconque des revendications 1 à 36, comprenant de plus une formulation pharmaceutique (22) qui contient un médicament et un excipient, la formulation pharmaceutique (22) convenant pour une application topique.

38. Dispositif de dosage (10) selon la revendication 37, dans lequel ladite formulation est un semi-solide, ledit semi-solide étant de préférence sélectionné dans l'ensemble constitué d'une pommade, d'un gel, d'une émulsion, d'une lotion, d'un spray, d'une crème et d'une pâte.

39. Dispositif de dosage (10) selon la revendication 37, dans lequel la formulation est un liquide, ledit liquide étant de préférence :
(a) une suspension ou une solution, ou
(b) converti en une mousse lors de l'actionnement et du déversement d'une dose entre ledit logement (21) et l'applicateur (30).

40. Dispositif de dosage (10) selon la revendication 37, dans lequel au moins environ 95 % dudit médicament sont absorbés par la peau en une durée inférieure à environ 30 minutes après administration, de préférence en une durée inférieure à environ 20 minutes après administration ou en une durée inférieure à environ 5 minutes après administration.

41. Dispositif de dosage (10) selon la revendication 37, dans lequel moins d'environ 50 % dudit médicament sont absorbés par la peau en une durée d'au moins environ 12 heures après administration, de préférence en une durée d'au moins environ 6 heures après administration ou en une durée d'au moins environ 2 heures après administration.

42. Dispositif de dosage (10) selon la revendication 37, dans lequel ledit médicament est sélectionné dans l'ensemble constitué des inhibiteurs d'ACE, des hormones adénohypophoséiques, des agents bloquants adrénergiques des neurones, de stéroïdes adrénocorticaux, d'inhibiteurs de la biosynthèse des stéroïdes adrénocorticaux, des agonistes alpha-adrénergiques, des antagonistes alpha-adrénergiques, des agonistes sélectifs alpha₂-adrénergiques, des analgésiques, des antipyrétiques, des agents anti-inflammatoires, des androgènes, des anesthésiants locaux, des anesthésiants généraux, des agents antiaddictifs, des antiandrogènes, des agents antiarrhythmiques, des agents antiasthmatiques, des agents anticholinergiques, des agents anticholinestérase, des anticoagulants, des agents antidiabétiques, des agents antidiarhée, des agents antidiurétiques, des agents antiémétiques, des agents prokinétiques, des agents antiépileptiques, des antioestrogènes, des agents antifongiques, des agents antihypertenseurs, des agents antimicrobiens, des agents antimigraines, des agents antimuscariniques, des agents antinéoplastiques, des agents antiparasites, des agents antiparkinson, des agents antiplaquettes, des agents antiprogestatifs, des agents antithyroïdiens, des agents antitussifs, des agents antiviraux, des antidépresseurs, des azaspirodécanediones, des barbituriques, des benzodiazépines, des benzothiadiazides, des agonistes bêta-adrénergiques, des antagonistes de b ê t a-adrénergiques, des antagonistes sélectifs de bêta₁-adrénergiques, des agonistes sélectifs de bêta₂-adrénergiques, des sels biliaires, des agents affectant le volume et la composition des fluides corporels, des butyrophénones, des agents affectant la calcification, des bloqueurs de canaux de calcium, des médicaments cardiovasculaires, des catécholamines, des médicaments sympathomimétiques, des agonistes cholinergiques, des réactivateurs de cholinestérase, des agents dermatologiques, des diphénylbutylpipéridines, des diurétiques, des alcaloïdes de l'ergot, des oestrogènes, des agents bloquant des ganglions, des agents stimulant les ganglions, des hydantoïnes, des agents de contrôle de l'acidité gastrique, des agents de traitement des ulcères peptiques, des agents hématopoïétiques, des antihistaminiques, des antagonistes de 5-hydroxytryptamine, des médicaments pour le traitement de l'hyperlipoprotéinémie, des hypnotiques, des sédatifs, des agents immunodépresseurs, des laxatifs, des bronchodilatateurs, des inhibiteurs de monoamine oxydase, des agents bloquants neuromusculaires, des nitrates organiques, des enzymes pancréatiques, des phénothiazines, des progestines, des prostaglandines, des agents antipsychotiques, des rétinoïdes, des bloqueurs de canaux sodiques, des agents anti-spasmes, des agents contre les spasmes musculaires aigus, des succinimides, des xanthines , des agents thrombolytiques, des agents de la thyroïde, des antidépresseurs tricycliques, des inhibiteurs du transport tubulaire des composés organiques, des médicaments affectant la motilité utérine, des vasodilatateurs et des vitamines, du bépridil, du diltiazem, du félodipine, de l'isradipine, de la nicardipine, de la nifédipine, de la nimodipine, de la nitrédipine, du vérapamil, de la dobutamine, de l'isoprotérénol, du cartérolol, du labétalol, du lévobunolol, du nadolol, du penbutolol, du pindolol, du propranolol, du sotalol, du timolol, de l'acébutolol, de l'aténolol, du bétaxolol, de l'esmolol, du métoprolol, de l'albutérol, du bitoltérol, de l'isoétharine, du métaprotérénol, du pirbutérol, de la ritodrine, de la terbutaline, de l'alclométasone, de l'aldostérone, de l'amcinonide, du béclométhasone, du dipropionate, du bétaméthasone, du clobétasol, du clocortolone, du cortisol, de la cortisone, de la corticostérone, du désonide, de la désoximétasone, de la 11-désoxycorticostérone, du 11-désoxycortisol, de la déxaméthasone, de la diflorasone, de la fludrocortisone, du flunisolide, de la fluocinolone, du fluocinonide, de la fluorométholone, du flurandrénolide, du halcinonide, de l'hydrocortisone, du médrysone, du 6.alpha-méthylprédnisolone, de la mométasone, de la paraméthasone, de la prédnisolone, de la prédnisone, du tétrahydrocortisol, de la triamcinolone, du benoxinate, de la benzocaïne, de la bupivacaïne, de la chloroprocaïne, de la cocaïne, de la dibucaïne, de la dyclonine, de l'étidocaïne, de la lidocaïne, de la mépivacaïne, de la pramoxine, de la prilocaïne, de la procaïne, de la proparacaïne, de la tétracaïne, de l'alfentanil, du chloroforme, de la clonidine, du dicyclopropane, du desflurane, du diéthyléther, du dropéridol, de l'enflurane, de l'étomidate, du halothane, de l'isoflurane, du chlorhydrate de cétamine, de la mépéridine, de la méthohexital, du méthoxyflurane, de la morphine, du propofol, du sevoflurane, du thiamylal, du thiopental, de l'acétaminophène, de l'allopurinol, de l'apazone, de l'aspirine, de l'auranofine, de l'aurothioglucose, de la colchicine, du diclofénac, du diflunisal, de l'étodolac, du fénoprofen, du flurbiprofen, du thiomalate de sodium et d'or, de l'ibuprofène, de l'indométacine, du kétoprofen, du méclofénamate, de l'acide méfénamique, de la mésélamine, du méthylsalicylate/salicylate de méthyl, de la nabumétone, du naproxène, de l'oxyphènebutazone, de la phénacétine, de la phénylbutazone, du piroxicam, du salicylamide, du salicylate, de l'acide salicylique, du salsalate, de la sulfasalazine, du sulindac, de la tolmétine, de l'acétophénazine, de la chlorpromazine, de la fluphénazine, de la mésoridazine, de la perphénazine, de la thioridazine, de la trifluorpérazine, de la triflupromazine, du disopyramide, de l'encaïnide, du flécaïnide, de l'indécaïnide, de la méxilétine, de la moricizine, de la phénytoïne, du procaïnamide, de la propafénone, de la quinidine, du tocaïnide, du cisapride, de la dompéridone, du dronabinol, de l'halopéridol, du métoclopramide, du nabilone, de la prochlorpérazine, de la prométhazine, de la thiéthylpérazine, du triméthobenzamide, de la buprénorphine, du butorphanol, de la dézocine, du diphénoxylate, du drocode, de l'hydrocodone, de l'hydromorphone, du levallorphan, du levorphanol, du lopéramide, du meptazinol, de la méthadone, de la nalbuphine, du nalméfène, de la nalorphine, de la naloxone, de la naltrexone, de l'oxybutynine, de la pentazocine, de l'isosorbide dinitrate, de la nitroglycérine, de la théophylline, de la phényléphrine, de l'éphidrine, de la pilocarpine, du furosémide, de la tétracycline, de la chlorphéniramine, du kétorolac, de la bromocriptine, du guanabenz, de la prazosine, de la doxazosine, de l'acide flufénamique et de leurs sels pharmaceutiquement acceptables, des benzodiazépines, telles que l'alprazolam, le brotizolam, de chlordiazépoxyde, du clobazam, du clonazépam, du clorazépate, du demoxépam, du diazépam, du flumazénil, du flurazépam, de l'halazépam, du lorazépam, du midazolam, du nitrazépam, du nordazépam, de l'oxazépam, du prazépam, du quazépam, du témazépam, du triazolam, et similaires; un agent antimuscarinique, par exemple de l'anisotropine, de l'atropine, du clidinium, du cyclopentolate, de la dicyclomine, du flavoxate, du glycopyrolate, de l'hexocyclium, de l'homatropine, de l'ipratropium, de l'isopropamide, du mépenzolate, de la méthanthéline, de l'oxyphéncyclimine, de la pirènzépine, de la propanthéline, de la scopolamine, de la télenzépine, du tridihéxéthyle, de la tropicamide, et similaires; et des oestrogènes par exemple du chlorotrianisène, du siéthylstilbestrol, du méthylestradiol, de l'estrone, de l'estrone de sulfate de sodium, de l'estropipate, du mestranol, du quinestrol, du sulfate d'équiline sodium, du 17.bêta.-estradiol (ou de l'estradiol), des dérivés d'oestrogènes semi synthétiques par exemple des esters of d'oestrogènes naturels comme du 17.bêta.-énanthate d'oestradiol, du 17.bêta.-valérate d'oestradiol, du 3-benzoate d'oestradiol, du 17.bêta.-undécénoate d'oestradiol, de l'oestradiol 16, du 17-hémisuccinate ou du 17.-bêta.-cypionate d'oestradiol et des oestrogènes 17-alkylés, comme l'éthynylestradiol, le 3-isopropylsulphonate d'éthyle-estradiol et similaires; des androgènes par exemple du danazol, de la fluoxymestérone, de la méthandrosténolone, de la méthyltestostérone, de la nandrolone descanoate, du phenpropionate de nadrolone, de l'oxandrolone, de l'oxymétholone, du stanozolol, de la testolactone, de la testostérone, du cypionate de testostérone, de l'enanthate de ctestostérone, du propionate de testostérone et similaires; ou une progestine telle que du diacétate d'éthynodiol, du gestodène, du caproate d'hydroxyprogestérone, du lévonorgestrel, de l'acétate de médroxyprogestérone, de l'acétate de mégestrol, de la noréthindrone, de l'acétate de noréthindrone, du noréthynodrel, du norgestrel, de la progestérone et de leurs sels pharmaceutiquement acceptables.

43. Dispositif de dosage (10) selon la revendication 37, dans lequel ledit médicament est un agent actif localement.

44. Dispositif de dosage (10) selon la revendication 43, dans lequel l'agent actif localement est présent pour l'utilisation dans le traitement de maladies de la peau sélectionnées dans l'ensemble constitué du psoriasis, de l'eczéma, de l'acné, de l'érythème, d'autres maladies inflammatoires, d'infections bactériennes, d'infections virales, d'infections fongiques, de conditions anaphylactiques, de malignités et de verrues.

45. Dispositif de dosage (10) selon la revendication 43, dans lequel l'agent actif est sélectionné dans l'ensemble constitué des anesthésiants, des corticostéroïdes, des agents antibactériens, des agents antifongiques ou de toutes leurs combinaisons thérapeutiquement efficaces, ledit anesthésiant étant de préférence sélectionné dans l'ensemble constitué de la bupivacaïne, de la lévo-bupivacaïne, de la ropivacaïne, de la benzocaïne, de la divucaïne, de la procaïne, de la chloroprocaïne, de la prilocaïne, de la mépivacaïne, de l'étidocaïne, de la tétracaïne, de la lidocaïne et de la xylocaïne ainsi que des dérivés actifs du point de vue de l'anesthésie, et de leurs analogues, isomères et mélanges.

46. Dispositif de dosage (10) selon la revendication 43, dans lequel l'agent actif localement est sélectionné dans l'ensemble constitué de la tétracaine, la benzocaine, la lindocaïne, l'hydrocortisone, la béclométhasone, le dipropionate, le propionate de clobétasol, le propionate de fluticasone, l'ichthammol, le succinate de lithium, le goudron de charbon, le dithranol, le peroxyde de benzoyle, la trétinoïne, le soufre, la vitamine D et les dérivés de ceux-ci, la framycétine, le chlorhydrate de chlortétracycline, l'acide fusidique, le clotrimazole, l'éconazole, l'amorolfine et la terbénafine, ou toute combinaison de ceux-ci efficace du point de vue thérapeutique.

47. Dispositif de dosage (10) selon la revendication 43, dans lequel l'agent actif localement est sélectionné parmi les agents antiviraux (par exemple de l'acyclovir et de l'idoxuridine, etc.), les agents antifongiques (par exemple de l'amphotéricine B, du clotrimazole, de la nystatine, du kétoconazole, du miconazole, du butocouazole, de l'haloprogine, etc.), les agents antibiotiques (les pénicillines, les céphalosporines, l'érythromycine, la tétracycline, la clindamycine, les aminoglycosides, le chloramphénicol, la polymixine b, la bacitracine, la néomycine, la gentamycine, etc.), les antiseptiques (par exemple de la povidone-iodine, du chlorure de méthylbenzéthonium, etc.), les antiparasites (par exemple du lindane, de l'anthraline, etc.), les agents analgésiques (par exemple du méthylsalicylate, de l'acide salicylique, de la dyclonine, de l'aloe vera, etc.), les anesthésiants locaux (par exemple de la benzocaïne, de la lidocaïne, de la xylocaïne, du picrate de butamben, etc.), les agents anti-inflammatoires (par exemple les composés contenant des stéroïdes tels que la dexaméthasone, la bétaméthasone, la prédnisone, la prédnisolone, la triamcinolone, l'hydrocortisone, l'alclométasone, l'amcinonide, la diflorasone, etc., ainsi que les non-stéroïdes anti-inflammatoires), des composés anti-gale et qui réduisent l'irritation (par exemple des antihistaminiques comme la diphénhydramine et les traitements du psoriasis), des composés soulageant les brûlures (par exemple du monoacétate d'o-amino-p-toluènesulfonamide, etc.), des agents de dépigmentation (par exemple du monobenzone) et des agents hormonaux (par exemple de l'oestriol).

48. Dispositif de dosage (10) selon la revendication 37, dans lequel ledit médicament présente une activité non locale.

49. Dispositif de dosage (10) selon la revendication 48, dans lequel ledit médicament est sélectionné parmi les vasodilatateurs, les substances actives pour le traitement d'une maladie du mouvement, des agents contraceptifs, des agents de remplacement d'hormone, des analgésiques et des aides pour arrêter de fumer ou toute combinaison thérapeutiquement efficace de ceux-ci, de la nitroglycérine, de la scopolamine, de l'estradiol, de la noréthistérone, du fentanyl et de la nicotine ou toute combinaison thérapeutiquement efficace de ceux-ci.

50. Dispositif de dosage (10) selon la revendication 37, dans lequel ledit excipient comprend un polymère, de préférence dans lequel ledit polymère est sélectionné dans l'ensemble constitué de l'alginate de sodium, de la gélatine, de l'amidon de maïs, de la gomme de tragacanthe, de la méthylcellulose, de l'hydroxyéthylcellulose, de la carboxyméthylcellulose, de la gomme de xanthan, de la dextrine, du carboxyméthylamidon, du poly(alcool vinylique), des polyacrylates de sodium, des copolymères d'anhydride maléique et de méthoxyéthylène, du poly(éther vinylique) et de la polyvinylpyrrolidone.

51. Dispositif de dosage (10) selon la revendication 37, dans lequel ledit excipient contient une cire, une graisse, une huile ou leurs combinaisons, graisse ou huile étant de préférence sélectionnée parmi la cire d'abeilles, l'huile d'olive, de beurre de cacao, l'huile de sésame, l'huile de soja, l'huile de camélia, l'huile d'arachides, la graisse de boeuf, le saindoux et la lanoline.

52. Dispositif de dosage (10) selon la revendication 37, dans lequel ledit excipient contient :
(a) de la vaseline blanche,
(b) une paraffine,
(c) un acide gras supérieur, de préférence l'acide stéarique,
(d) un alcool supérieur, de préférence sélectionné dans l'ensemble constitué de l'alcool de cétyle, de l'alcool de stéaryle et de leurs combinaisons,
(e) un polyéthylène glycol ou
(f) de l'eau.

53. Dispositif de dosage (10) selon la revendication 37, dans lequel ledit médicament contient de l'oxyde d'azote.

54. Dispositif de dosage (10) selon l'une quelconque des revendications 1 à 52, pour administrer de façon topique une formulation pharmaceutique (22) sur la peau d'un mammifère, le dispositif (10) étant actionné et la dose étant appliquée directement sur la peau du mammifère avec l'applicateur (30).

55. Dispositif de dosage (10) selon la revendication 54, dans lequel le dispositif peut être actionné une deuxième fois et des doses supplémentaires de ladite formulation pharmaceutique (22) peuvent être administrées.

56. Dispositif de dosage (10) selon la revendication 54, dans lequel ledit médicament :
(a) a un effet local sur la surface de la peau,
(b) est absorbé et a un effet local dans la région d'application ou
(c) est absorbé et a un effet systémique.

57. Procédé de préparation d'un système de dosage pour application topique d'une formulation pharmaceutique (22) comprenant les étapes qui consistent à :
(i) préparer au moins une dose d'une préparation pharmaceutique comprenant un médicament incorporé dans un excipient pharmaceutiquement acceptable qui convient pour une application topique sur la peau dudit mammifère et
(ii) placer au moins une dose dans un dispositif de dosage selon la revendication 1.
